(19) Europäisches Patentamt / European Patent Office / Office européen des brevets

(11) **EP 4 105 329 A1**

(12) **EUROPEAN PATENT APPLICATION**

(43) Date of publication:
**21.12.2022 Bulletin 2022/51**

(21) Application number: **21180165.9**

(22) Date of filing: **17.06.2021**

(51) International Patent Classification (IPC):
**C12N 15/113** $^{(2010.01)}$    **A61P 25/00** $^{(2006.01)}$

(52) Cooperative Patent Classification (CPC):
**C12N 15/1138;** C12N 2310/20

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Eberhard Karls Universität Tübingen Medizinische Fakultät**
**72074 Tübingen (DE)**

(72) Inventors:
• **Marciano, Sabina**
  **72076 Tübingen (DE)**

• **Pichler, Bernd**
  **85298 Scheyern (DE)**
• **Herfert, Kristina**
  **72074 Tübingen (DE)**

(74) Representative: **Witte, Weller & Partner Patentanwälte mbB**
**Postfach 10 54 62**
**70047 Stuttgart (DE)**

Remarks:
The complete document including Reference Table(s) and the Sequence Listing(s) can be downloaded from the EPO website

(54) **COMPOUND AND METHOD FOR THE TREATMENT OF A VMAT2-ASSOCIATED DISEASE**

(57)    The invention is directed to a 'single guide RNA' (sgRNA) molecule for use in the prophylaxis and/or treatment and/or examination of a disease, preferably a VMAT2-associated disease, a vector comprising said sgRNA, a composition comprising said vector, a nucleic acid molecule, and methods associated therewith.

EP 4 105 329 A1

**Description**

The invention is directed to a 'single guide RNA' (sgRNA) molecule for use in the prophylaxis and/or treatment and/or examination of a disease, preferably a VMAT2-associated disease, a vector comprising said sgRNA, a composition comprising said vector, a nucleic acid molecule, and methods associated therewith.

FIELD OF THE INVENTION

The present invention relates to the field of molecular biology and molecular medicine, more particular to the field of genetic engineering applications, preferably to the targeted editing of disease-associated genes.

BACKGROUND OF THE INVENTION

The mammalian brain is a network of spatially distributed but functionally and structurally interconnected regions that exhibit correlated activity over time. They communicate with each other via highly specialized neuronal connections, and are organized in neuronal circuits and networks. Understanding how functional connections between regions are organized in the healthy and diseased brain is therefore of great interest.

Genetic manipulations of neuronal subtypes have revealed valuable information on functional brain circuits and their relationships to individual phenotypes. Genome engineering methodologies based on CRISPR/associated RNA-guided endonuclease (Cas9) has enabled researchers to interrogate the mammalian DNA in a precise yet simple manner. CRISPR/Cas9 represents a promising approach to unveil the influence of genes on brain circuits, and its potential in manipulating the genome has been ascertained in several species. CRISPR/Cas9 has been shown to precisely edit genes of single or multiple genomic loci *in vitro* and *in vivo*. However, one great hurdle is the brain delivery, which must comply with effective nuclear access, while minimizing immunogenic reactions and off-target editing.

Resting-state fMRI (rs-fMRI) has enabled neuroscientists to delineate the level of functional communication between anatomically separated regions. Rs-fMRI measures the resting-state functional connectivity (rs-FC) at high spatial and temporal resolutions based on spontaneous fluctuations at rest of the blood oxygen level-dependent (BOLD) signal, which indirectly detects neuronal activity via hemodynamic coupling. Using rs-fMRI several brain resting-state networks in humans and rodents have been identified, such as the default mode and sensorimotor networks (DMM, SMN). These networks are altered in neurological diseases, meaning they could serve as therapeutic and diagnostic biomarkers. However, the underlying molecular changes driving the pathological alterations remain largely unknown.

Positron emission tomography (PET) provides a non-invasive tool to indirectly measure neurotransmitter changes in the brain with high specificity, identifying the focus of abnormalities. One well characterized example is the radioligand [$^{11}$C]raclopride (RAC), a widely used D2/D3 receptor antagonist, enabling the non-invasive determination of dopamine availability and release.

PET combination with BOLD-fMRI has great potential for the *in vivo* investigation of neurotransmitter systems. Only a few studies used simultaneous PET/fMRI, enabling the direct spatial and temporal correlation of both measurements. In this context, the inventors have recently shown that resting state functional connectivity is modulated by intrinsic serotonin transporter and D2/3 receptor occupancy in rats.

Multiple line of evidence suggests a broader role of dopamine in the dynamic reconfiguration of functional brain networks, underlying pathological patterns of disease. Despite advances in science and medicine, the understanding of the dopaminergic modulation of the brain function and a targeted treatment of disorders related to dopaminergic function or neuropsychiatric disorders remain unsatisfactorily resolved.

Against this background it is an object underlying the invention to provide a new compound which can be used for a targeted prophylaxis and/or treatment and/or examination of a disease, preferably disorders related to dopaminergic function or neuropsychiatric disorders. Preferably, such a compound should be provided which can be used as a gene editing tool, for instance within the frame of the CRISPR/Cas9 technology.

The present invention satisfies these and other needs.

SUMMARY OF THE INVENTION

The present invention provides a 'single guide RNA' (sgRNA) molecule targeting the *Slc18a2* gene in a living being for use in the prophylaxis and/or treatment and/or examination of a disease, characterized in that the sgRNA molecule comprises a nucleotide sequence which is selected from the group consisting of SEQ ID NOS: 1-5.

According to the invention 'single guide RNA' (sgRNA) or guide RNA is a component of the CRISPR complex which serves to guide the CRISPR endonuclease Cas9 to its target, i.e. the *Slc18a2* gene. An sgRNA is a non-coding short ribonucleic acid (RNA) sequence which binds to the complementary target DNA sequence. The sgRNA guides the CRISPR endonuclease enzyme Cas9 to the specific *Slc18a2* gene location on the DNA, where the CRISPR endonuclease Cas9 mediates a double-strand break.

According to the invention a '*Slc18a2* gene' or 'solute carrier family 18 member A2' gene (human variant: HGNC: 10935; NCBI Entrez Gene: 6571; Ensembl: ENSG00000165646; rat variant: NCBI Entrez Gene: 25549, Ensembl: ENSRNOG00000008890) encodes a transmembrane protein that functions as an ATP-dependent transporter of monoamines, such as dopamine, norepinephrine, serotonin, and histamine. The coding product is also referred to as 'vesicular monamine transporter 2' (VMAT2). This protein transports amine neurotransmitters into synaptic vesicles. Polymorphism in this gene have been reported as being associated with schizophrenia, bipolar disorder, and other neurological/psychiatric ailments.

According to the invention a 'living being' includes any beings comprising a *Slc18a2* gene, including mammals, preferably rats and human beings.

The inventors were surprisingly successful in editing or knocking down the *Slc18a2* gene, *in vivo* and *in vitro,* by using the sgRNA molecule according to the invention in the context of the CRISPR/Cas9 technology. Thereby, VMAT2 has been inactivated. Even more surprisingly, neuronal integrity was not negatively affected by this approach. Further, toxicity effects associated with the sgRNA according to the invention were at a minimum. The inventors, therefore, provide for the first time a valuable molecular tool which can be successfully used in the prophylaxis and treatment of genetic diseases, preferably such diseases associated with VMAT2. It turned out that the indicated nucleotide sequences, especially SEQ ID NO: 1, were particularly effective in editing the *Slc18a2* gene while others tested by the inventors were not. The invention is also useful for the preclinical screening of novel therapeutic interventions for dopaminegic-dysfunction diseases.

Therefore, in an embodiment of the sgRNA according to the invention said disease is associated with the 'vesicular monoamine transporter 2' (VMAT2).

The type 2 vesicular monoamine transporter (VMAT2), by regulating the storage of monoamines transmitters into synaptic vesicles, has a protective role against their cytoplasmic toxicity. Increasing evidence suggests that impairment of VMAT2 neuroprotection contributes to the pathogenesis of several diseases associated with VMAT2. Among such diseases the following are to be named explicitly: disorders related to dopaminergic function (i.e. diseases characterized in dopamine imbalance), neuropsychiatric disorders (i.e. disorders of affect, cognition, and behavior that arise from overt disorder in cerebral function, or from indirect effects of extracerebral disease), Parkinson's disease (PD), movement disorders, Huntington's disease, tardive dyskinesia schizophrenia, and addiction.

Against this background, in an embodiment of the indentation said VMAT2-associated disease is selected from the group consisting of: disorders related to dopaminergic function, neuropsychiatric disorders, Parkinson's disease (PD), movement disorders, Huntington's disease, tardive dyskinesia schizophrenia, and addiction.

Another subject-matter of the invention relates to a vector comprising the sgRNA molecule according to the invention.

According to the invention, a 'vector' includes any DNA molecule used as a vehicle to artificially carry the sgRNA into another cell, where it can be replicated and/or expressed. Viral vectors are of particular preference as they are characterized by embodying the viruses' specialized molecular mechanisms to efficiently transport genomes inside the cells they infect.

The features, characteristics, advantages and embodiments described for the sgRNA according to the invention also apply to the vector according to the invention.

In a preferred embodiment of the invention the vector according to vector is an adeno-associated vector (AAV), preferably with AAV-PHP.EB serotype.

This measure has the advantage that such a vector is used which, when using the CRISPR/Cas9 technology, results in particularly high spreading of the CRISPR/Cas9 components, hence superior gene editing. Given the translational potential of CRISPR/Cas9, its brain delivery through AAVs may hold great promises for gene therapy of neurological disorders. In this regard, the generation of novel viral vector capsids permeating the blood brain barrier (BBB) represents a unique opportunity to overcome the obstacle of BBB penetration. The most efficient vector serotype of this series is the AAV-PHP.EB, an enhanced version of its precursor capsid AAV-PHP.B, showing higher brain tropism and transduction rate; see Seo, J.W., et al., Positron emission tomography imaging of novel AAV capsids maps rapid brain accumulation. Nat Commun, 2020. 11(1): p. 2102; Chatterjee, D., et al., Enhanced CNS transduction from AAV.PHP.eB infusion into the cisterna magna of older adult rats compared to AAV9. Gene Ther, 2021. The AAV-PHP.EB serotype is also suitable for local vector delivery. Indeed, it leads to focal expression of the desired vector 4 weeks post-injection in the rat *substantia nigra* (SN), with an efficiency significantly higher compared to other serotypes; Dayton, R.D., et al., More expansive gene transfer to the rat CNS: AAV PHP.EB vector dose-response and comparison to AAV PHP.B. Gene Ther, 2018. Another feature of the AAV-PHP.EB is its ability to efficiently transduce neurons with multiple vectors at the time; Chan, K.Y., et al., Engineered AAVs for efficient noninvasive gene delivery to the central and peripheral nervous systems. Nat Neurosci, 2017. 20(8): p. 1172-1179. Recently, it has been used to drive the expression of CRISPR/Cas9-components; see Seo, J.W., et al. (*l.c.*). For all of the above reasons the choice of using the AAV.PHP.EB capsid also broadens the applicability of the sgRNA according to the invention.

In a further embodiment of the vector according to the invention the sgRNA is under the control of an U6 promoter.

The U6 promoter driving the sgRNA expression in CRISPR/Cas9 vectors shows superior editing efficiency; see Wei, Y., et al., CRISPR/Cas9 with single guide RNA expression driven by small tRNA promoters showed reduced editing efficiency compared to a U6 promoter. RNA, 2017. 23(1): p. 1-5. CRISPR/Cas9 vector systems using the U6 promoter to drive sgRNA expression have also been successfully used in the rat brain; see Sun, H., et al., Development of a CRISPR-SaCas9 system for projection- and function-specific gene editing in the rat brain. Sci Adv, 2020. 6(12): p. eaay6687.

Another subject-matter of the invention relates to a composition comprising the vector according to the invention.

The features, characteristics, advantages and embodiments described for the sgRNA and the vector according to the invention also apply to the composition according to the invention.

In an embodiment of the invention said composition further comprises a vector encoding CRISPR associated protein 9 (Cas9), preferably *Staphylococcus aureus* Cas9 (SaCas9).

This measure establishes the requirements for applying the CRISPR/Cas9 technology. Cas9 (CRISPR associated protein 9, formerly called Cas5, Csn1, or Csx12), a 160 kilodalton protein, refers to an enzyme which cleaves the phosphodiester bond within a polynucleotide chain, which is a component of a CRISPR (Clustered Regularly Interspaced Short Palindromic Repeat) complex. The Cas9 is, therefore, capable to specifically bind to the sgRNA which directs the endonuclease to the target nucleic acid. *Staphylococcus aureus* CRISPR Cas9 (SaCas9) is a small Cas9 ortholog and can be packed in AAVs, overcoming their packaging constraints. SaCas9 effective gene targeting has been validated in adult rats; see Sun, H., et al., (*I.c.*). Other smaller Cas9 have been recently discovered, such as originating from *Staphylococcus auricularis* or *Campylobacter jejuni* or *Neisseria meningitidis,* and are likewise suitable; see Hu, Z., et al., A compact Cas9 ortholog from Staphylococcus Auricularis (SauriCas9) expands the DNA targeting scope. PLoS Biol, 2020. 18(3): p. e3000686; Kim, E., et al., In vivo genome editing with a small Cas9 orthologue derived from Campylobacter jejuni. Nat Commun, 2017. 8: p. 14500; Ibraheim, R., et al., All-in-one adeno-associated virus delivery and genome editing by Neisseria meningitidis Cas9 in vivo. Genome Biol, 2018. 19(1): p. 137.

In an embodiment of the composition according to the invention said Cas9 is under the control of a CAG promoter.

The CAG (CMV enhancer/chicken β-Actin promoter) promoter-driven expression is superior over other promoters, especially the common synapsin's promoter in the long-term; see Jackson, K.L., et al., Better Targeting, Better Efficiency for Wide-Scale Neuronal Transduction with the Synapsin Promoter and AAV-PHP.B. Front Mol Neurosci, 2016. 9: p. 116. This aspect is crucial in the treatment, prophylaxis or modelling of neurodegenerative disease, which require long-term expression/monitoring. CAG has been the promoter of choice in the latest studies employing AAV.PHP.EB; see Seo, J.W., et al. (*I.c.*).

In another embodiment of the invention said composition is a pharmaceutical composition comprising a pharmaceutically acceptable carrier.

A "pharmaceutical composition" is a composition suitable for an administration to an animal and/or human being in a medical setting. Preferably, a pharmaceutical composition is sterile and produced according to GMP guidelines.

'Pharmaceutically acceptable carriers' or excipients are well known in the art and include, for example, nanocarriers, nanovectors, aqueous solutions such as water or physiologically buffered saline or other solvents or vehicles such as glycols, glycerol, oils such as olive oil or injectable organic esters. In preferred embodiments, when such pharmaceutical compositions are for human administration, e.g., for parenteral administration, the aqueous solution is pyrogen-free, or substantially pyrogen-free. The excipients can be chosen, for example, to effect delayed release of an agent or to selectively target one or more cells, tissues or organs. The pharmaceutical composition can be in dosage unit form such as an injection, tablet, capsule (including sprinkle capsule and gelatin capsule), granule, powder, syrup, suppository, or the like. The composition can also be present in a solution suitable for topical administration. Alternatively, the pharmaceutical composition can be present in form of an aerosol administrable by inhalation.

Suitable pharmaceutical carriers or excipients as well as pharmaceutical necessities for use in pharmaceutical formulations are described in Remington - The Science and Practice of Pharmacy, 23rd edition, 2020, a well-known reference text in this field, and in the USP/NF (United States Pharmacopeia and the National Formulary). Other sources are also available to one of ordinary skill in the art.

The pharmaceutical composition as well as the methods to be explained below of the present invention may be utilized to treat a living being in need thereof. In certain embodiments, the living being is a mammal such as a human, rat or another non-human mammal.

In an embodiment to the composition according to the invention the latter is configured for the prophylaxis and/or treatment and/or examination of a disease associated with the 'vesicular monoamine transporter 2' (VMAT2), preferably for the prophylaxis and/or treatment and/or examination of a disease associated with the 'vesicular monoamine transporter 2' (VMAT2).

Another subject-matter of the invention relates to a nucleic acid molecule, preferably an RNA molecule, further preferably an sgRNA molecule, said molecule comprising a nucleotide sequence which is selected from the group consisting of SEQ ID NOS: 1-5.

A 'nucleic acid molecule' as referred to herein includes both DNA and RNA molecules comprising natural and/or chemically modified nucleotides.

The features, characteristics, advantages and embodiments described for the sgRNA according to the invention also apply to the nucleic acid molecule according to the invention.

Another subject-matter of the invention relates to a method *in vitro* for editing the *Slc18a2* gene in biological material including genetic material encoding said *Slc18a2* gene, comprising the step of introducing the sgRNA according to the invention, and/or said vector according to the invention, and or said composition according to the invention, and/or said nucleic acid molecule according to the invention into said biological material, preferably said editing is made via CRISPR/Cas9 technology.

According to the invention 'gene editing' or genomic editing is a type of genetic engineering in which DNA is inserted, deleted, modified or replaced in the genome of a living organism. In the context of the invention 'gene editing' refers to the modification of the *Slc18a2* gene. In an embodiment of the invention the gene editing results in a targeted knockout a specific functional elimination of the expression of the *Slc18a2* gene, respectively.

According to the invention, 'biological material including genetic material encoding said *Slc18a2* gene' includes biological cells, tissues and parts of organisms.

Another subject-matter according to the invention relates to a method for the prophylaxis and/or treatment and/or examination of a disease associated with 'vesicular monoamine transporter 2' (VMAT2) in a living being, comprising a step of editing the *Slc18a2* gene in said living being by introducing the sgRNA according to the invention, and/or said vector according to the invention, and or said composition according to the invention, and/or said nucleic acid molecule according to the invention into said living being, preferably said gene editing is made via CRISPR/Cas9 technology.

In an embodiment of the method according to the invention said disease is selected from the group consisting of: disorders related to dopaminergic function, neuropsychiatric disorders, Parkinson's disease (PD), movement disorders, Huntington's disease, tardive dyskinesia, schizophrenia, and addiction.

The features, characteristics, advantages and embodiments described for the sgRNA, vector, composition, and nucleic acid molecule according to the invention also apply to the methods according to the invention.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

BRIEF DESCRIPTION OF THE FIGURES

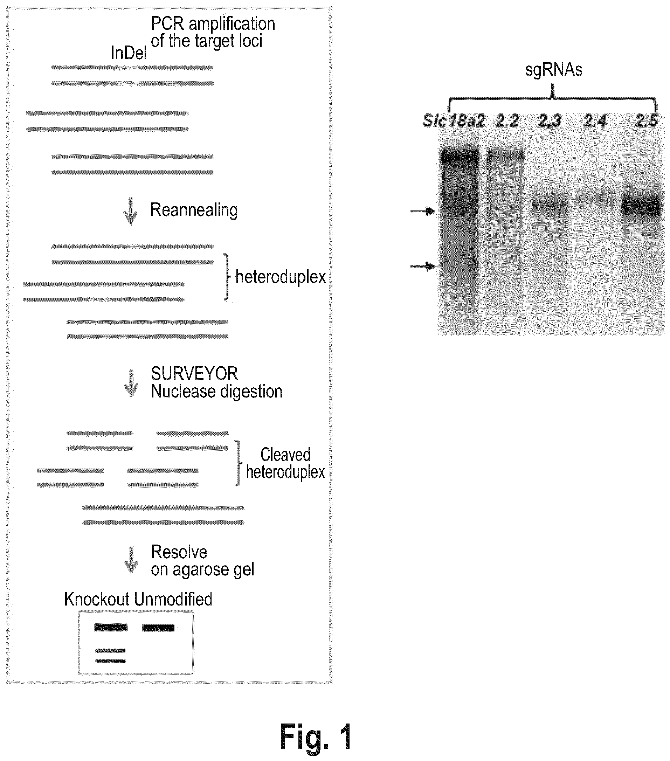

Fig. 1: Left: Surveyor assay workflow: DNA from primary neurons was extracted and the sequence of interest was amplified with PCR. Following amplica-tion of the target locus, re-annealing of the mutated and wild-type se-quences was carried out. The re-annealing converts the mutations into mismatch duplexes (heteroduplexes), which can be recognized perform-ing an enzymatic digestion. To display the occurring InDels, the digested product is then resolved on agarose gel. Right: Agarose gel is displayed. Arrows indicate the expected cleavage products only for the sgRNA-*Slc18a2*.

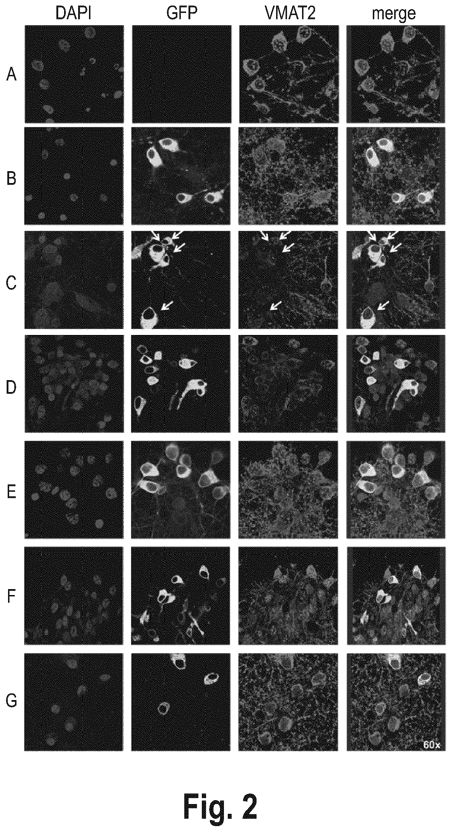

Fig. 2: VMAT2 (red) immunofluorescence in cultured neurons 7 days post-trans-duction: a: Non-transduced neurons. b: Neurons transduced with SaCas9, control sgRNA (targeting bacterial LacZ gene). c-g: Neurons transduced with SaCas9 and the selected sgRNAs targeting the *Slc18a2* locus. Nuclei were labeled with DAPI (blue). GFP signal (green) indicates for sgRNA vectors expression. White arrows indicate transduced neurons character-ized by reduced VMAT2 staining (red).

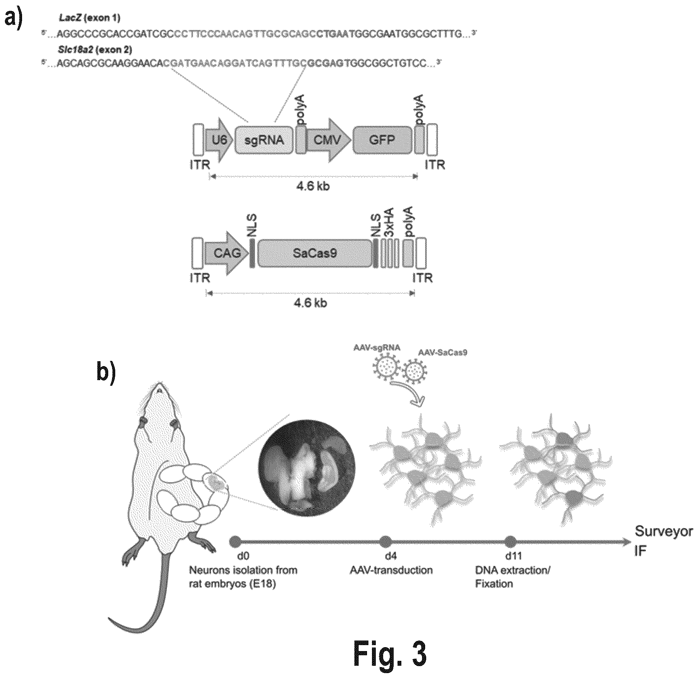

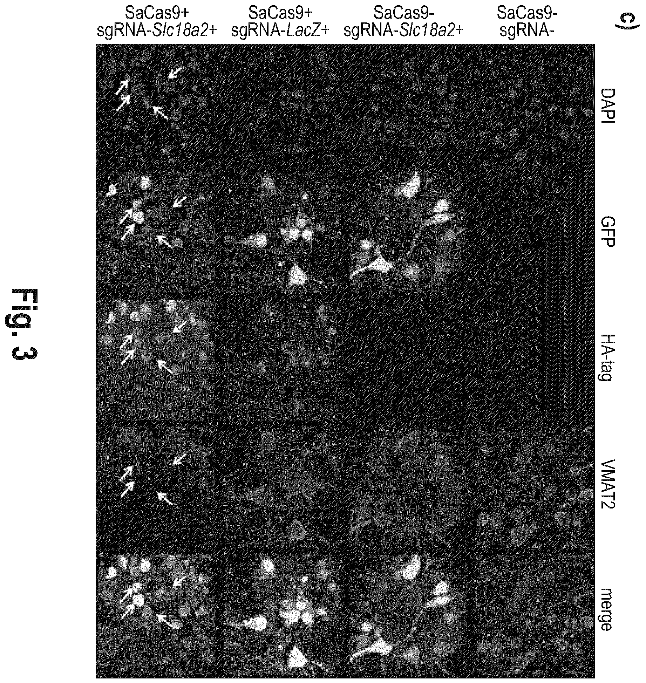

Fig. 3: *In vitro* study design and validation of the CRISPR/SaCas9-induced VMAT2 KD. a, AAV-sgRNA vectors encode, under the U6 promoter, the sgRNAs targeting the loci of interest for *LacZ* (control, exon 1) or *Slc18a2* (VMAT2 KD, exon 2). The vectors express GFP via the CMV promoter to allow the visualization of targeted neurons. AAV-SaCas9 vector encodes the expression of the nuclease flanked by two NLS to allow its transloca-tion into the nuclei, under the control of the CAG promoter. The construct contains three HA-tags for the identification of transduced neurons. CMV: cytomegalovirus, GFP: green fluorescent protein, CAG: CMV en-hancer/chicken β-Actin promoter, NLS: nuclear localization sequence, HA-tag: hemagglutinin. b, Embryos (E18) were isolated from the mother rat and decapitation was performed. Neurons were dissected from the cortices and transduced with the AAVs for sgRNA and SaCas9 expres-sion. One-week post-transduction, neurons were processed for the IF. c, VMAT2 staining (red) was performed in non-transduced neurons, neurons transduced with AAV-sgRNA-*Slc18a2*, neurons transduced with AAV-SaCas9 and AAV-sgRNA-LacZ, and neurons receiving AAVs for the SaCas9 and sgRNA-*Slc18a2* expression. Nuclei were labeled with DAPI (blue). GFP (green) and HA-tag (white) signals indicate the expression of the sgRNA and SaCas9 vectors, respectively. The inventors observed a detectable reduction of the VMAT2 protein (arrows) in neurons transduced with AAVs carrying SaCas9 and sgRNA-*Slc18a2*.

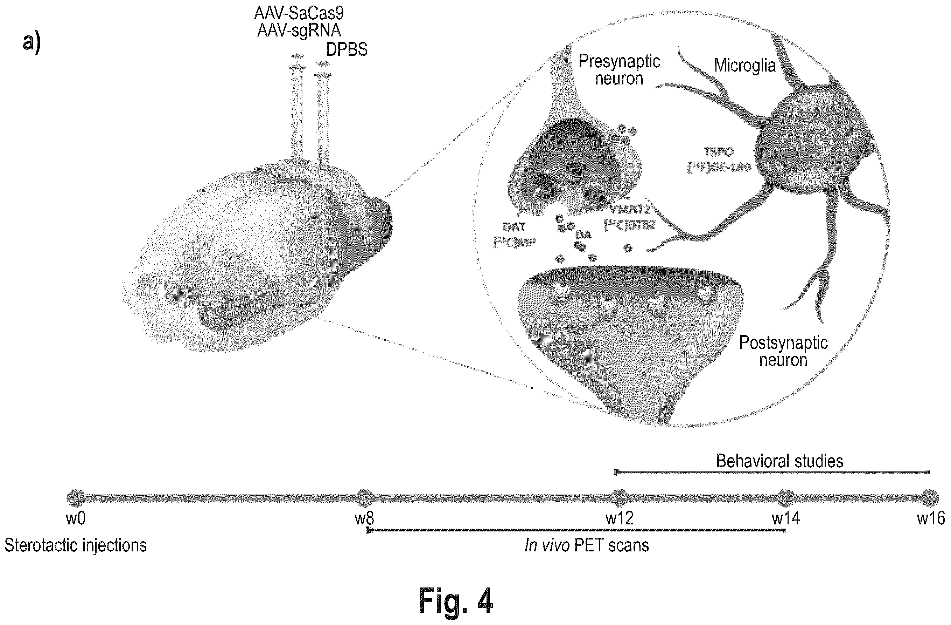

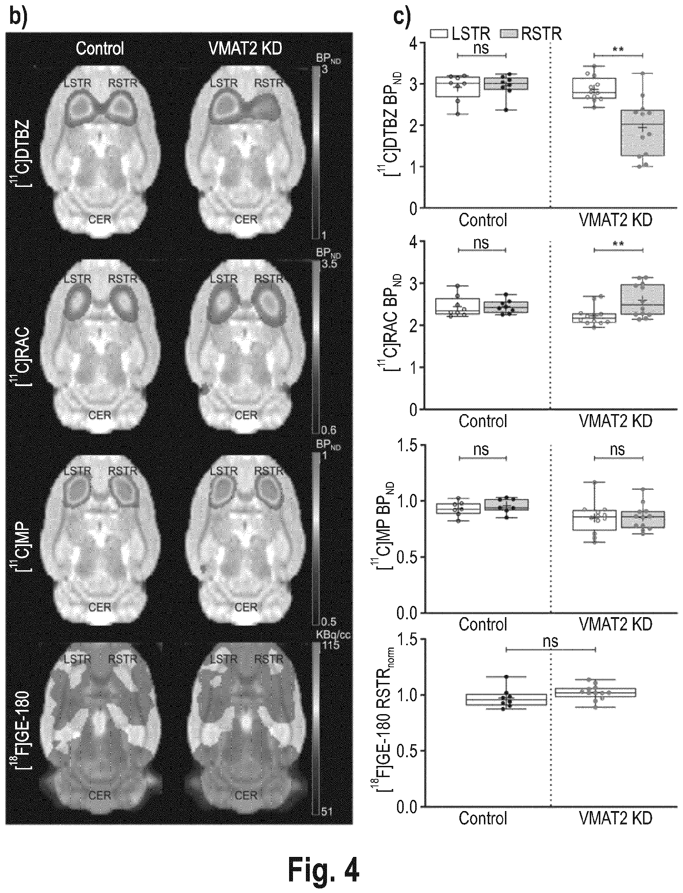

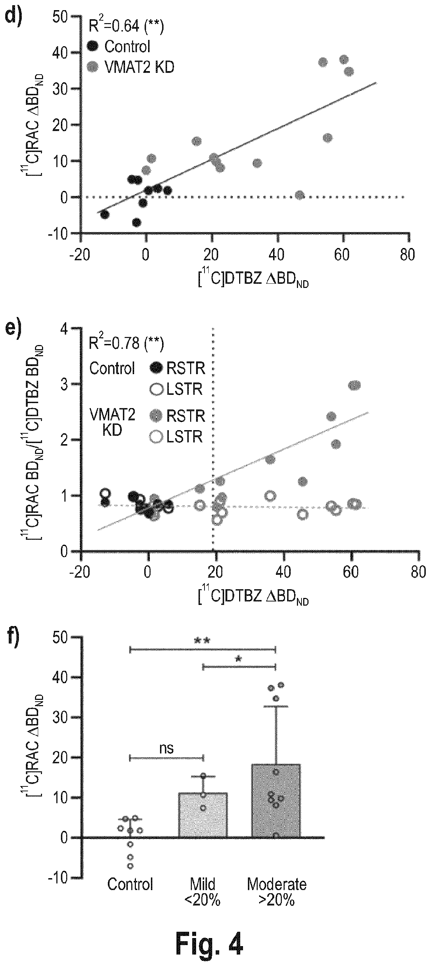

Fig. 4: *In vivo* PET quantification of the CRISPR/SaCas9-induced VMAT2 KD. a, AAVs for SaCas9 and sgRNA-*Slc18a2* (VMAT2 KD) or sgRNA-LacZ (con-trol) were injected into the right SNc of wild-type rats. DPBS was injected into the contralateral SNc. Starting 8 weeks post-injection the CRISPR/SaCas9-induced changes of VMAT2, DAT and DAR expression, and microglia activation were quantified *in vivo* with [11C]DTBZ, [11C]MP, [11C]RAC and [18F]GE-180, respectively. 12 weeks after CRISPR/Cas9 targeting the rats' motor function was assessed performing the cylinder, open field, beam walk and rotameter tests. b, [11C]DTBZ, [11C]RAC, [11C]MP and [18F]GE-180 voxel-wise coronal $BP_{ND}$ maps images (mean images) of control and VMAT2 KD rats, co-registered to the Schiffer rat brain atlas. c, Graphs showing radioligands $BP_{ND}$ in the left (white bar) and right (grey bar) striatum of control (black) and VMAT2 KD (red) rats. $BP_{ND}$ were calculated from individual TACs of the left striatum, right stria-tum (target regions) and cerebellum (reference region), and quantified with Logan reference. [18F]GE-180 SUVs of the right striatum were calcu-lated over the interval between 30 and 60 min after scan start and normal-ized by the SUVs calculated for the left striatum over the same interval. The inventors observed a reduction of [11C]DTBZ $BP_{ND}$ in the right stria-tum of VMAT2 KD (*n*= 12; **$P$= 0.0003), but not control rats (*n*= 8; *ns P*= 0.54). [11C]RAC data showed an increased $BP_{ND}$ in the right stria-tum of VMAT2 KD rats (*n*= 12; **$P$= 0.001), and no differences in the stri-ata of control rats (*n*= 8; *ns P*= 0.97). The CRISPR/SaCas9-induced VMAT2 KD did not affect [11C]MP binding, as no differences were ob-served between the striata of both control (*n*= 7; *ns P*= 0.06) and VMAT2 KD rats (*n*= 12; *ns P*= 0.82). The inventors did not observe changes in [18F]GE-180 SUVs (*ns P*= 0.17) between control (*n*= 8) and VMAT2 KD rats (n= 12), implying that no glial activation occurred following the CRISPR/SaCas9-mediated VMAT2 KD. d, The inventors observed a great correlation between [11C]RAC $\Delta BP_{ND}$ and [11C]DTBZ $\Delta BP_{ND}$ ($R^2$= 0.64, **$P$< 0.0001), and a fine separation between groups. e, Ratio of striatal [11C]RAC $BP_{ND}$ and [11C]DTBZ $BP_{ND}$ evidenced prominent [11C]RAC changes when ~ 20% [11C]DTBZ $\Delta BP_{ND}$ is reached (Left striatum: $R^2$= 0.02, *ns P*= 0.59; right striatum: $R^2$= 0.78, **$P$< 0.0001). This threshold was therefore set to separate the VMAT2 KD rats into mild and moderate. Empty and full dots correspond to left and right striatum, re-spectively. g, Rats were separated in control (*n*= 8), mild (*n*= 3) and mod-erate (*n*= 9), depending on the level of synaptic dysfunction estimated from [11C]DTBZ $\Delta BP_{ND}$. [11C]RAC would discriminate between different de-grees of synaptic disfunction (Control vs mild *ns P*= 0.07, mild vs moderate *$P$= 0.004, control vs moderate **$P$< 0.0001). Each boxplot describes the median value (central mark), the mean value (plus sign), interquartile range (boxes edges), and the extreme points from the distribution (whiskers). Single-subject data are also plotted over each boxplots as col-oured circles (black or red for control or VMAT2 KD, respectively). Paired t-test was used for the statistical comparison between striata. One-way Anova multiple comparisons was used for the lesion discrimination analy-sis. Correlations plots were obtained from linear regression analysis. Re-sults were considered significant for +$P \leq$ 0.05, *$P \leq$ 0.01, **$P \leq$ 0.001 and *ns* for $P$> 0.05. Control: AAV-SaCas9 and AAV-sgRNA-*LacZ*; VMAT2 KD: AAV-SaCas9 and AAV-sgRNA-*Slc18a2*. LSTR: left striatum, RSTR: right striatum, CER: cerebellum.

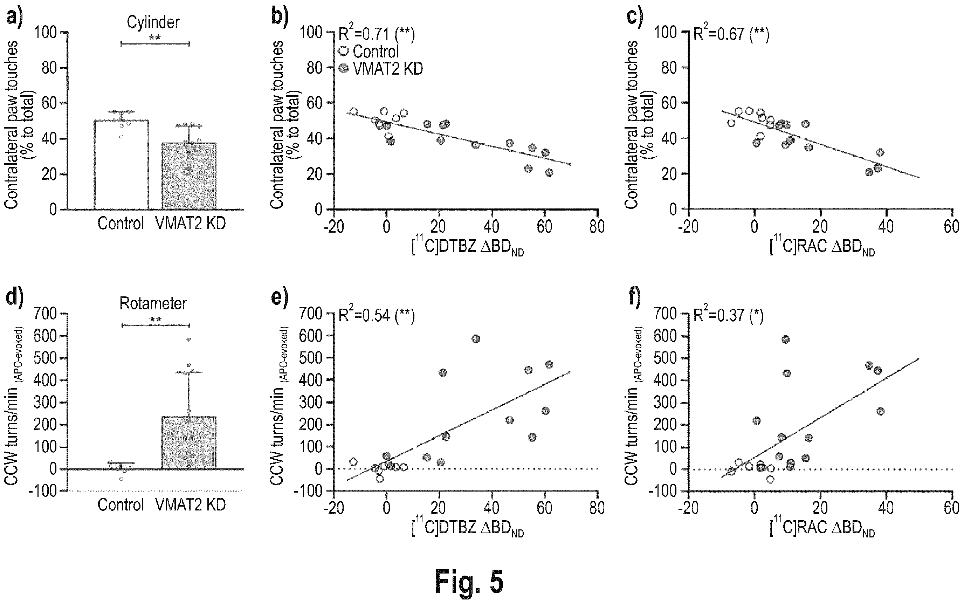

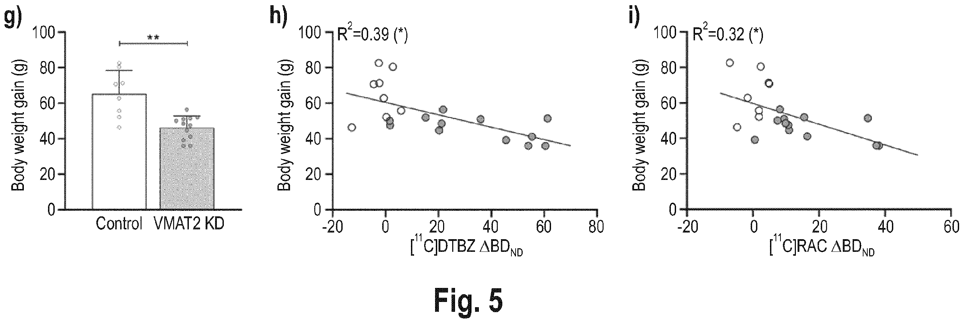

Fig. 5: Behavioral assessment of the CRISPR/SaCas9-induced VMAT2 KD. a, VMAT2 KD rats (*n*= 12) showed a reduction in the contralateral paw touches, compared to controls (*n*= 8) in the cylinder test (**$P$= 0.002). b,c, Rats performance in the cylinder test strongly correlated with [11C]DTBZ $\Delta BP_{ND}$ ($R^2$= 0.71, **$P$< 0.0001) and [11C]RAC $\Delta BP_{ND}$ ($R^2$= 0.67, **$P$< 0.0001). d, Following apomorphine administration, VMAT2 KD rats (*n*= 12) displayed a higher number of CCW rotations compared to control rats (*n*= 8) in the rotameter test (**$P$= 0.0042). e,f, Apomorphine-induced rotations exhibited a strong correlation with [11C]DTBZ ($R^2$= 0.54, **$P$= 0.0002) and [11C]RAC binding changes ($R^2$= 0.37, *$P$= 0.005). g, A reduction in body weight gain was observed in VMAT2 KD (*n*= 12) but not control rats (*n*= 8) 14 weeks after CRISPR/SaCas9 editing (**$P$= 0.0004). h,i, Body weight gain correlated with [11C]DTBZ $\Delta BP_{ND}$ ($R^2$= 0.39, *$P$= 0.003) and [11C]RAC $\Delta BP_{ND}$ ($R^2$= 0.32, *$P$= 0.01). Each bar describes the mean $\pm$ SD. Single-subject data are also plotted over each bar as col-oured circles (white for control rats, grey for VMAT2 KD rats). Unpaired t-test was used. Correlations plots were obtained from linear regression analysis. Results were considered significant for $^+P \leq 0.05$, $^*P \leq 0.01$, $^{**}P \leq 0.001$ and *ns* for $P > 0.05$. Control: AAV-SaCas9 and AAV-sgRNA-*LacZ*; VMAT2 KD: AAV-SaCas9 and AAV-sgRNA-*Slc18a2*.

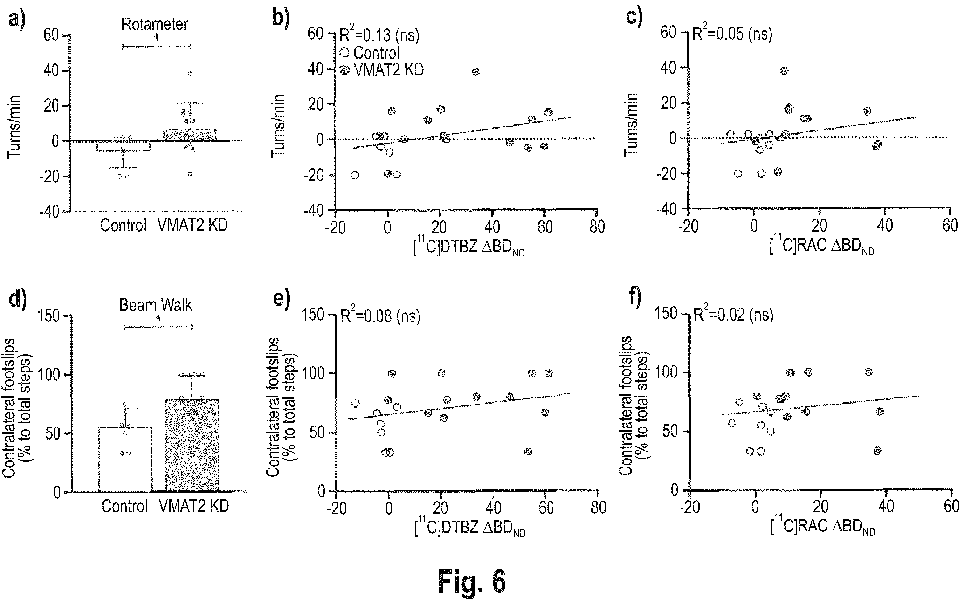

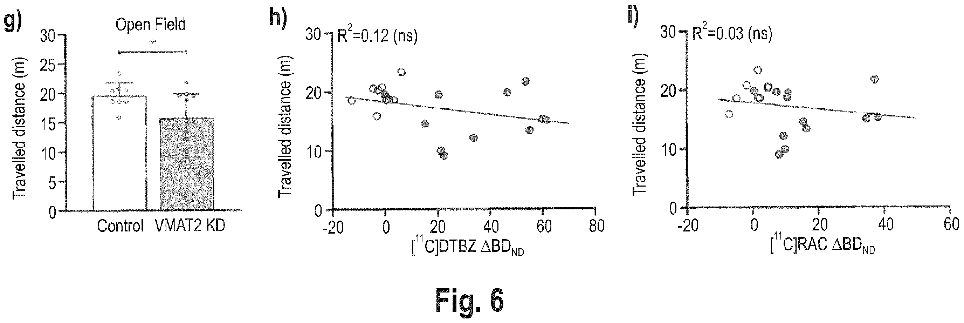

Fig. 6: Behavioral assessment of the CRISPR/SaCas9-induced VMAT2 KD. a, Spontaneous rotation in a novel spherical environment evidenced in-creased ipsilateral turns in VMAT2 KD rats (*n* = 12) compared to controls (*n* = 8) ($^+P$ = 0.05). b, c, Spontaneous rotations did not correlate with [$^{11}$C]DTBZ $\Delta BP_{ND}$ ($R^2$ = 0.13, *ns P* = 0.12) and [$^{11}$C]RAC $\Delta BP_{ND}$ ($R^2$ = 0.05, *ns P* = 0.33). d, VMAT2 KD rats (*n* = 12) displayed a higher number of footslips (falls) to the left contralateral side compared to control rats (*n* = 8) while performing the beam walk task ($^*P$ = 0.01). e, f, No correlations be-tween gait alterations and [$^{11}$C]DTBZ $\Delta BP_{ND}$ ($R^2$ = 0.08, *ns P* = 0.24), or [$^{11}$C]RAC $\Delta BP_{ND}$ ($R^2$ = 0.02, *ns P* = 0.51) were found. g, The inventors ob-served a reduction in the distance travelled (m) by VMAT2 KD rats (*n* = 12) in the open field test, compared to control rats (*n* = 8) ($^+P$ = 0.03). h, i, We identified no correlation between locomotor activity and [$^{11}$C]DTBZ $\Delta BP_{ND}$ ($R^2$ = 0.11, *ns P* = 0.14), and [$^{11}$C]RAC $\Delta BP_{ND}$ ($R^2$ = 0.03, *ns P* = 0.44). Each bar describes the mean $\pm$ SD. Single-subject data are also plotted over each bar as coloured circles (white for control rats, grey for VMAT2 KD rats). Unpaired t-test was used. Correlations plots were obtained from lin-ear regression analysis. Results were considered sig-nificant for $^+P \leq 0.05$, $^*P \leq 0.01$, $^{**}P \leq 0.001$, and *ns* for $P > 0.05$. Control: AAV-SaCas9 and AAV-sgRNA-*LacZ*; VMAT2 KD: AAV-SaCas9 and AAV-sgRNA-*Slc18a2*.

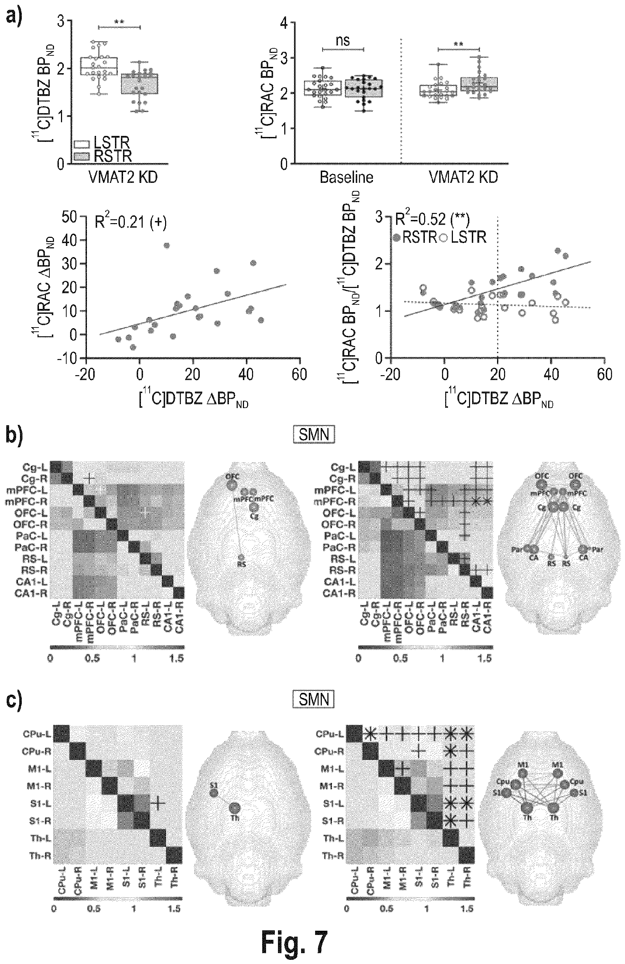

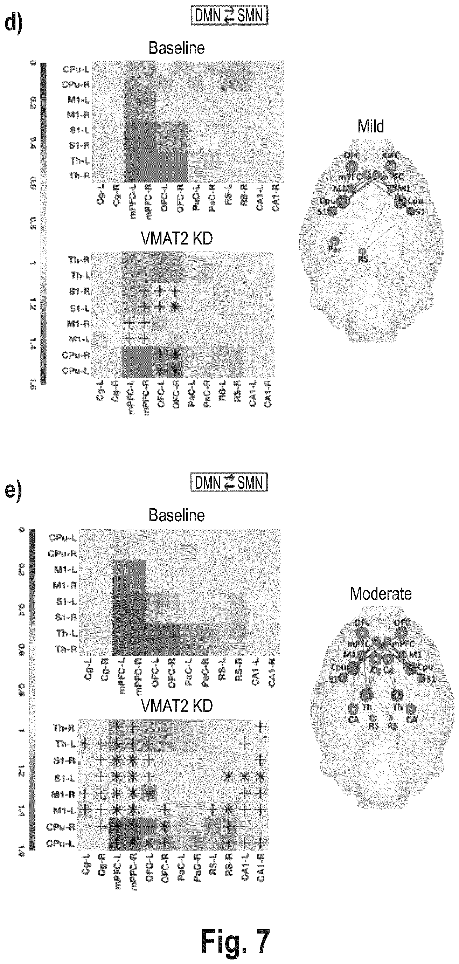

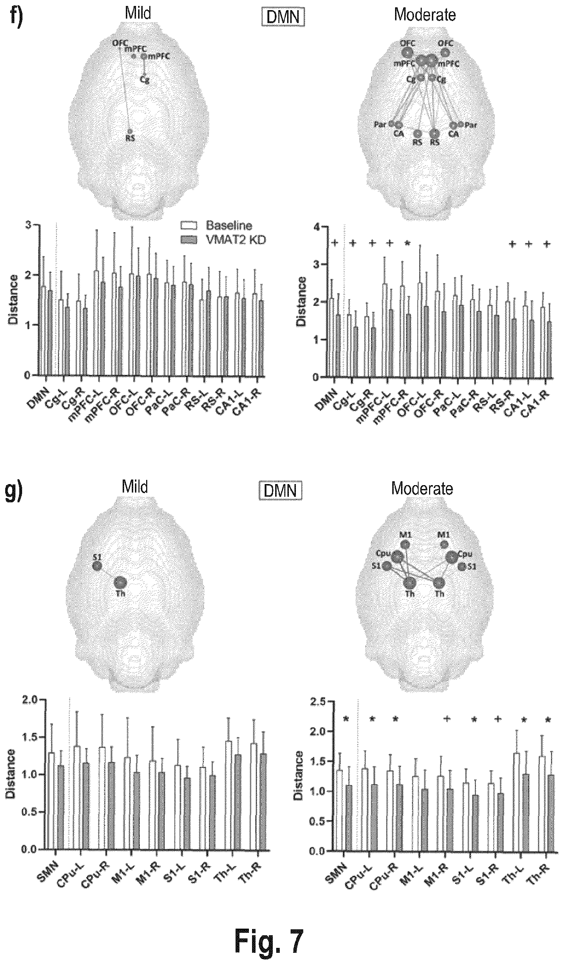

Fig. 7: CRISPR/SaCas9 gene-editing and [$^{11}$C]RAC PET/fMRI. a, Graphs show-ing [$^{11}$C]DTBZ and [$^{11}$C]RAC $BP_{ND}$ in the left (white bar) and right (grey bar) striatum. $BP_{ND}$ were calculated from individual TACs of the left stria-tum, right striatum (target regions) and cerebellum (reference region), and quantified with Logan reference. The inventors observed a reduction of [$^{11}$C]DTBZ $BP_{ND}$ in the right striatum of VMAT2 KD rats (*n* = 23; $^{**}P <$ 0.0001). [$^{11}$C]RAC data showed an increased $BP_{ND}$ in the right stria-tum of VMAT2 KD rats (*n* = 23; $^{**}P$ = 0.0001), and no differences in the stri-ata of rats scanned at baseline (n = 23; *ns P* = 0.39). The inventors identi-fied a strong correlation between [$^{11}$C]RAC $\Delta BP_{ND}$ and [$^{11}$C]DTBZ $\Delta BP_{ND}$ ($R^2$ = 0.21, $^+P$ = 0.03). The ratio of striatal [$^{11}$C]RAC $BP_{ND}$ and [$^{11}$C]DTBZ $BP_{ND}$ evidenced an increase into the right striatum (Left striatum: $R^2$ = 0.02, *ns P* = 0.49; right striatum: $R^2$ = 0.52, $^{**}P <$ 0.0001). Prominent [$^{11}$C]RAC $BP_{ND}$ changes occurred when $\sim$ 20% [$^{11}$C]DTBZ $\Delta BP_{ND}$ is reached. According to this threshold VMAT2 KD rats were separated into mild (*n* = 13) and moderate (*n* = 10). Each boxplot describes the median value (central mark), the mean value (plus sign), interquartile range (boxes edges), and the extreme points of the distribution (whiskers). Single-subject data are also plotted over each boxplots as coloured circles (black for baseline, red for VMAT2 KD). Empty and full dots correspond to left and right striatum, respectively. Group level correlation matrices of the DMN (b) and SMN (c) at baseline (below matrix diagonal) and after VMAT2 KD (above matrix diagonal) for mild and moderate rats. Brain graphs, right to the matrices, illustrate the nodes and edges (raw values) that demonstrated FC changes to baseline (%) in DMN (b) and SMN (c). b, In VMAT2 KD mild rats the inventors observed FC changes only in re-gions of the anterior DMN (mPFC-L and OFC, mPFC-R and Cg), showing a conversing FC pattern. In VMAT2 KD moderate rats the inventors ob-served FC increase in most regions of the anterior DMN, extending to the posterior DMN (RS bilaterally, RS-R and CA1 bilaterally). The inventors found low within-DMN FC changes in mild rats, but strong within-DMN FC changes in moderate rats. Interestingly, a decreased FC within OFC-L and RS was found in mild but not moderate VMAT2 KD rats. c, In VMAT2 KD mild rats the inventors observed increased FC throughout S1-L and Th-L. In VMAT2 KD moderate rats FC activation intensified throughout S1-L and Th-L, and extended to most of the regions of the SMN. The in-ventors found low within-SMN FC changes in mild cases, but strong within-SMN FC changes in moderate cases. d, Internetwork FC changes in VMAT2 KD mild rats, compared to baseline, indicated increased FC be-tween anterior regions of the DMN and the SMN (between OFC and CPu, S1 bilaterally, between mPFC and M1, S1 bilaterally), and decreased FC between posterior regions of the DMN and the SMN (between RS-L and M1-R, and S1 bilaterally). e, Increased DMN-SMN FC was found in VMAT2 KD moderate rats, compared to baseline. Internetwork FC alter-ations did not occur between regions of the posterior DMN which showed deactivation in the mild subjects, but extended to Cg, CA1, and S1, M1, Th. f,g, Graph theoretical analyses of the global mean connection dis-tance indicated no changes, compared to baseline, for VMAT2 KD mild rats in the DMN and SMN. Instead, VMAT2 KD moderate rats presented shorter functional paths to the nodes. Interestingly, while network organi-zation changes affected bilaterally the regions of the DMN (f), they oc-curred to a higher extent in the left regions of the SMN (g). Each bar de-scribes the mean value $\pm$ SD. Paired t-tests were used. Correlations plots were obtained from linear regression analysis. Results were considered significant for $^+P \leq 0.05$, $^*P \leq 0.01$, $^{**}P \leq 0.001$ and *ns* for $P > 0.05$. Baseline: wild-type LE rats; VMAT2 KD: AAV-SaCas9 and AAV-sgRNA-*Slc18a2*. Mild: [$^{11}$C]DTBZ $\Delta BP_{ND} <$ 20%; Moderate: [$^{11}$C]DTBZ $\Delta BP_{ND} >$ 20%. LSTR: striatum, RSTR: right striatum, CER: cerebellum. DMN: default-mode network, SMN: sensorimotor network.

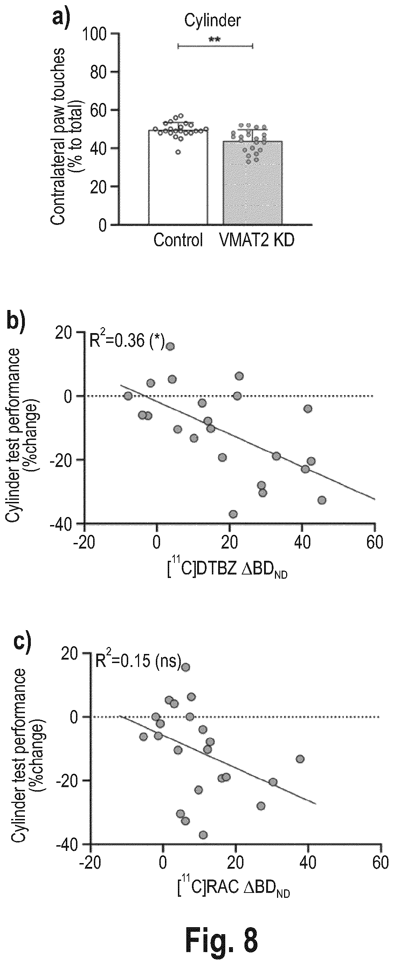

Fig. 8: Behavioral assessment of the CRISPR/SaCas9-induced VMAT2 KD in the PET/fMRI study cohort. a, Cylinder test was performed in LE rats ($n$= 23) at baseline and 12 weeks after CRISPR/SaCas9-targeting. VMAT2 KD rats showed a reduction in the contralateral paw touches, compared to baseline (**$P$= 0.001). b, c, Rats performance in the cylinder test, % change from baseline, correlated with [11C]DTBZ $\Delta BP_{ND}$ ($R^2$= 0.36, *$P$= 0.003), but not with [11C]RAC $\Delta BP_{ND}$ ($R^2$= 0.15, $ns$ $P$= 0.07). Each bar describes the mean $\pm$ SD. Single-subject data are also plotted over each bar as coloured circles (white for baseline, grey for VMAT2 KD). Unpaired t-test was used. Correlations plots were obtained from linear regression analysis. Results were considered significant for $^+P\leq$ 0.05, *$P\leq$ 0.01, **$P\leq$ 0.001, and $ns$ for P> 0.05. Baseline: wild-type LE rats; VMAT2 KD: AAV-SaCas9 and AAV-sgRNA-$Slc18a2$.

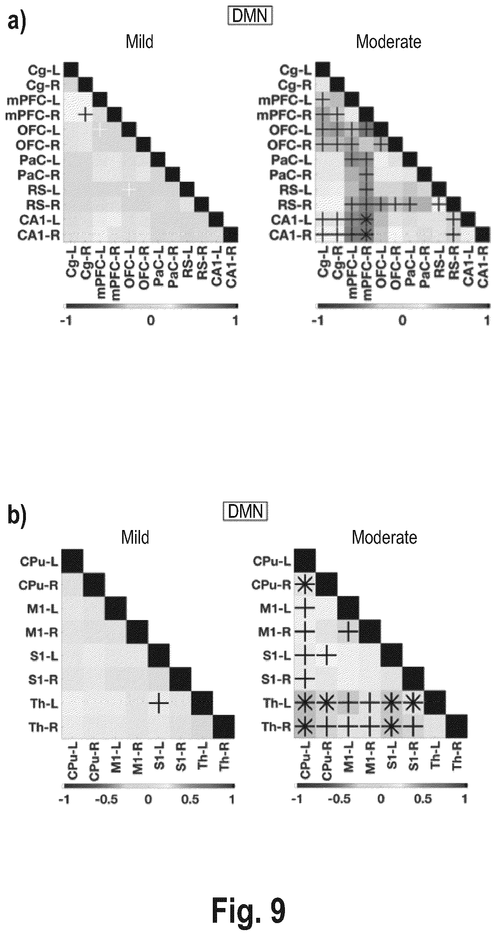

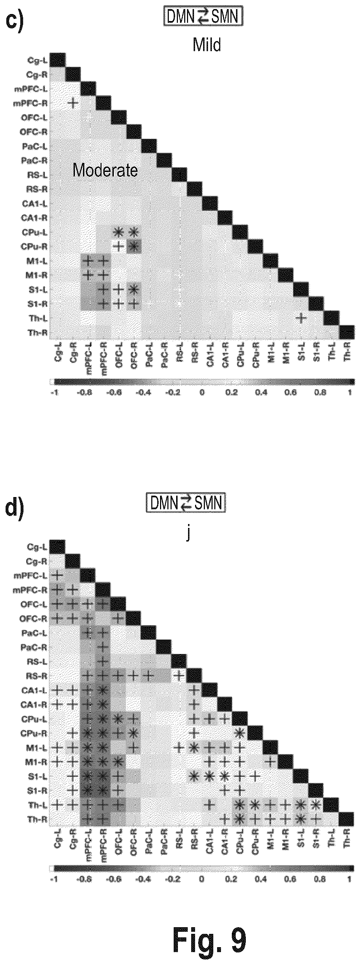

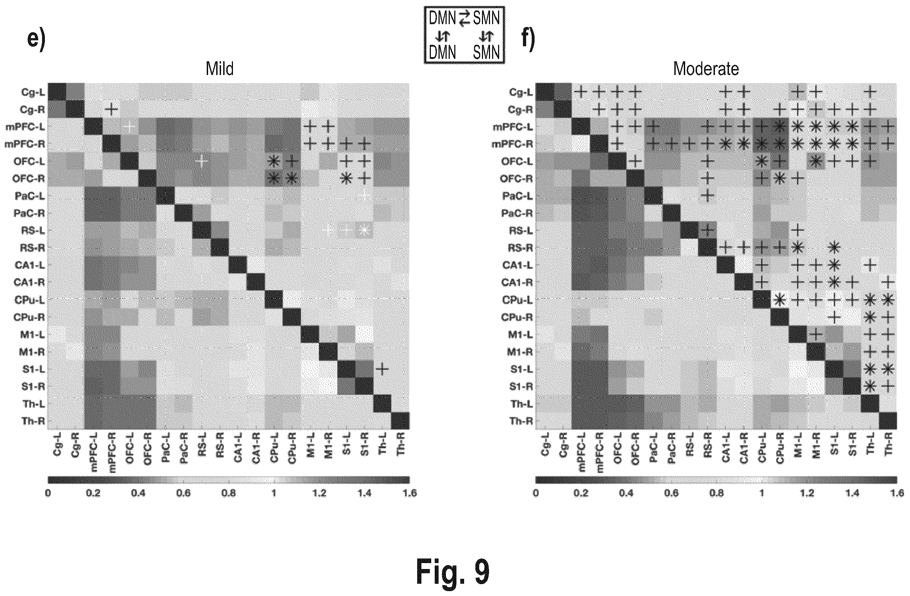

Fig. 9: CRISPR/SaCas9 gene-editing and [11C]RAC PET/fMRI a,b, Group level correlation matrices illustrate the FC changes to baseline (%) for VMAT2 KD mild and moderate rats in DMN and SMN, respectively. c, FC changes to baseline (%) for VMAT2 KD mild rats within and between DMN and SMN. d, FC changes to baseline (%) for VMAT2 KD moderate rats within and between DMN and SMN. e,f, Within-network and internetwork FC al-terations are displayed in a group level correlation matrix. In the above matrix diagonal, FC changes for VMAT2 KD mild (e) and moderate (f) rats are reported, compared to the baseline (below matrix diagonal). Paired t-tests were used. Results were considered significant for $^+P\leq$ 0.05, *$P\leq$ 0.01, **$P\leq$ 0.001 and $ns$ for P> 0.05. Baseline: wild-type LE rats; VMAT2 KD: AAV-SaCas9 and AAV-sgRNA-$Slc18a2$. Mild: [11C]DTBZ $\Delta BP_{ND}$ < 20%; Moderate: [11C]DTBZ $\Delta BP_{ND}$ > 20%. DMN: default-mode network, SMN: sensorimotor network.

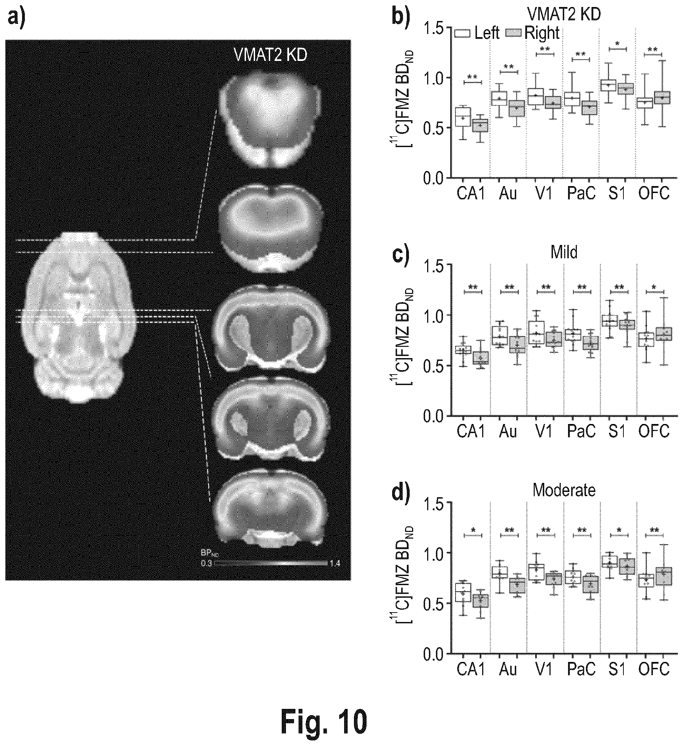

Fig. 10: CRISPR/SaCas9 gene-editing and $in$ $vivo$ PET imaging of DA-GABA in-terplay. a, [11C]FMZ voxel-wise coronal $BP_{ND}$ maps images (mean im-ages) of VMAT2 KD rats, co-registered to the Schiffer rat brain atlas. To the right panel, graphs for [11C]FMZ $BP_{ND}$ in the left (white bar) and right (grey bar) CA1, Au, V1, PaC, S1, and OFC of VMAT2 KD rats are dis-played. b, [11C]FMZ $BP_{ND}$ for the complete cohort (n= 23). c, [11C]FMZ $BP_{ND}$ for VMAT2 KD mild rats ($n$= 13). d, [11C]FMZ $BP_{ND}$ for VMAT2 KD moderate rats (n= 10). $BP_{ND}$ were calculated from individual TACs of the target regions, and pons (reference region), and quantified with Logan ref-erence. The inventors observed a reduction of [11C]FMZ $BP_{ND}$ in the right CA1 (**$P$= 0.001), Au (**$P$= 0.001), V1 (**$P$= 0.001), PaC (**$P$= 0.001), S1 (*$P$= 0.003), and an increase of [11C]FMZ $BP_{ND}$ in the right OFC (**$P$= 0.001) of VMAT2 KD rats (n= 23). Results were not conditional on the degree of synaptic dys-function, estimated from [11C]DTBZ $\Delta BP_{ND}$, as they were consistent between VMAT2 KD mild and moderate rats. Each boxplot describes the median value (central mark), the mean value (plus sign), interquartile range (boxes edges), and the extreme points from the distribution (whiskers). Single-subject data are also plotted over each box-plots as circles. Paired t-tests were used. Bonferroni-Sidak corrected P values are shown. Results were considered significant for $^+P \leq$ 0.05, *$P \leq$ 0.01, **$P \leq$ 0.001 and $ns$ for P> 0.05. VMAT2 KD: AAV-SaCas9 and AAV-sgRNA-$Slc18a2$. Mild: [11C]DTBZ $\Delta BP_{ND}$ < 20%; Moderate: [11C]DTBZ $\Delta BP_{ND}$ > 20%. CA1: hippocampus, Au: auditory cortex, V1: Vis-ual cortex, PaC: parietal cortex, S1: somatosensory cortex, OFC: or-bitofrontal cortex.

EXAMPLES

1. Material and methods

*Animals*

Female wild-type Long Evans (LE) rats (224 $\pm$ 30 g, n= 55, Charles River Laboratories, Sulzfeld, Germany) were kept on a 12 h day-night cycle at a room temperature of 22 °C and 40 - 60% humidity. Animals received a standard diet and tap water $ad$ $libitum$ before and during the experimental period. All animal experiments were performed according to the German Animal Welfare Act and were approved by the local ethical authorities, permit numbers R15/19M, R4/20G.

*Viral vectors*

SgRNAs targeting the second exon of the $Slc18a2$ gene (VMAT2 KD) and the first exon of the $LacZ$ gene (control) were designed based on the PAM sequence of SaCas9 (NNGRRT; SEQ ID NO: 7) (Table 1).

Table 1: sgRNA sequences

| sgRNAs Sequences | DNA Target Sequences 5'-3' | Designed SgRNAs 5'-3' | PAM (NNGRRT; SEQ ID NO: 7) 5'-3' |
|---|---|---|---|
| Slc18a2 | CGATGAACAGGATCAGTTTGC (SEQ ID NO: 8) | GCAAACTGATCCTGTTCATCG (SEQ ID NO: 1) | GCGAGT (SEQ ID NO: 14) |
| Slc18a2.2 | CAGCGCAAGGAACACGATGAA (SEQ ID NO: 9) | TTCATCGTGTTCCTTGCGCTG (SEQ ID NO: 2) | CAGGAT (SEQ ID NO: 15) |
| Slc18a2.3 | CTTAATGCTGTACAGATAGCT (SEQ ID NO: 10) | AGCTATCTGTACAGCATTAAG (SEQ ID NO: 3) | GGGGAT (SEQ ID NO: 16) |
| Slc18a2.4 | GTCTGGTGGTCTGGATTTCCG (SEQ ID NO: 11) | CGGAAATCCAGACCACCAGAC (SEQ ID NO: 4) | TAGAGT (SEQ ID NO: 17) |
| Slc18a2.5 | GAACTCTACTGTGTTGATCACC (SEQ ID NO: 12) | GGTGATCAACACAGTAGAGTTC (SEQ ID NO: 5) | GGGAAT (SEQ ID NO: 18) |
| LacZ | CCTTCCCAACAGTTGCGCAGC (SEQ ID NO: 13) | GCTGCGCAACTGTTGGGAAGG (SEQ ID NO: 6) | CTGAAT (SEQ ID NO: 19) |

SgRNAs were cloned into an AAV-PHP.EB expression vector containing a GFP reporter sequence, driven by the CMV promoter, for the identification of transduced neurons. A second AAV-PHP.EB construct was produced to express SaCas9, flanked by two NLS to allow its translocation into the nuclei. The vector expressed the nuclease via the CAG promoter, and presented three HA-tags to visualize the targeted neurons (Fig. 3a). Cloning of the sgRNAs, plasmid construction, as well as the production of concentrated and purified AAV-PHP.EB vectors, were carried by SignaGen Laboratories (Rockville, USA) following the inventors' indications for the vectors' design. AAVs deliver-able grade was ~ $10^{13}$ gc/mL. Vectors were tittered to the desired working concentration ~ $10^{12}$ gc/mL, as quantified through qPCR, to perform *in vivo* and *in vitro* experiments.

*Rat primary cortical neuron culture*

Primary cortical neurons were obtained from rat embryos of a pregnant Sprague Dawley rat from embryonic day 18 (E18) (Charles River, Sulzfeld, Germany). Embryos were decapitated and quickly removed from the mother rat. Cortical dissection was performed in ice-cold HBSS (100 mL 10 x HBSS, 870 mL dH$_2$O, 3.3% 0.3 M HEPES pH 7.3 and 1% pen/strep) (LifeTechnologies, Massachusetts, USA). The obtained tissue was washed three times with 10 mL ice-cold HBSS PhenolRed-free (LifeTechnologies, Massachusetts, USA) and then digested at 37 °C for 20 min in 8 mL HBSS with 2.5% trypsin (LifeTechnologies, Massachusetts, USA). Cortices were washed 3 times with 10 mL HBSS containing 3.7% FBS, and then gently triturated in 2 mL HBSS. For the maintenance, neurons were plated on poly-d-lysine-coated 24 well plates (BD Biosciences, Heidelberg, Germany) or coverslips (Neuvitro Corporation, Vancouver, USA) at a density of 16 x $10^4$/well, and cultured in Neurobasal media supplemented with 2% 1 x B27, 0.25% Glutamax, 0.125% Glutamate and 1% pen/strep (LifeTechnologies, Massachusetts, USA) for four days. Afterwards, AAVs carrying the expression of the vectors for the SaCas9 and sgRNA (1:1 ratio), were added to the culture medium at 200,000 MOI (average titer 1.7 x $10^{12}$ gc/mL, final viral volume 37.5 μL/well). Neurons were processed 1 week post-viral treatment (Fig. 3b).

*Surveyor assay*

To estimate the VMAT2 KD efficiency of the designed sgRNA *in vitro,* we evaluated the presence of genetic deletions in rat primary neurons with the Surveyor assay (Surveyor kit, Integrated DNA Technologies, Coralville, USA). One week following the viral infection the genomic DNA was extracted using the QuickExtract DNA Extraction solution (Epicentre, Madison, USA), according to the manufacturer's instructions, and was normalized to 100 ng in dH$_2$O. 18 - 25 nt primers were designed 200 - 400 bp away from either side of the SaCas9 target site in order to amplify the loci of interest by touchdown PCR (oligonucleotides used for PCR are provided in Supplementary Table 1).

| sgRNAs | Forward Primer 5'-3' | Reverse Primer 5'-3' | Fragment size (bp) |
|---|---|---|---|
| *Slc18a2* | CTTGGGGATCCTCTAAGGCAG (SEQ ID NO: 20) | TACAGCGCGGTTCTTCAACT (SEQ ID NO: 22) | 313_280 |
| *LacZ* | GTCGTGACTGGGAAAACCCT (SEQ ID NO: 21) | TTGTTCCCACGGAGAATCCG (SEQ ID NO: 23) | 200_80 |
| Supplementary Table 1: Primers used to amplify the *Slc18a2* and the *LacZ* loci in the Surveyor assay and DNA expected fragments sizes | | | |

DNA amplification was performed using 0.5 μL Phusion Polymerase (LifeTecologies, Massachusetts, USA), as previously reported. A single band product was visualized on 1.5% agarose gel, isolated and purified using QuiaQuick Spin columns (Qiagen, Hilden, Germany), following the supplier protocol. 400 ng of the purified PCR product were mixed with 2 μL Taq DNA polymerase buffer (LifeTechnologies, Massachusetts, USA) in order to allow the cross-annealing of the mutated and wild-type sequences. The re-annealing process was conducted at the following cycling conditions: 10 min at 95 °C, 95 °C to 85 °C at -2 °C/s, hold 1 min, ramp down to 75 °C at -0.3 °C/s, hold 1 min, and so on until 4 °C temperature was reached. This cross-annealing procedure converts the mutations into mismatch duplexes (heteroduplexes), which can be recognized performing a nuclease digestion. The inventors digested the annealed products for 20 min at 42 °C using 2.5 μL MgCl$_2$ (0.15 M), 1 μL Surveyor nuclease and 1 μL Surveyor enhancer. Digested products were then resolved on a 2.5% agarose gel, stained with 1% SYBR Gold DNA (LifeTechnologies, Massachusetts, USA) in 1% TBE buffer. The size of the occurring bands indicated the location of the mutation (expected DNA fragments sizes are provided in Supplementary Table 1). To quantify the KD efficiency, the software ImageJ was used. Peak areas of the bands visualized on agarose gel were selected and the percentages of the transduced neurons acquiring the InDel mutation were calculated using the following formula:

$$InDel\ (\%) = 100 \ \times (1 - \sqrt{\left(1 - \frac{b+c}{a+b+c}\right)}$$

where *a* is the integrated intensity of the undigested PCR product and *b* and c are the integrated intensities of each cleavage fragment from the digested product.

*Immunofluorescence of rat primary neurons*

Rat primary neurons were processed 1 week post AAV-transduction. Coverslips were washed twice with DPBS and fixed in 4% PFA in DPBS for 15 min at room temperature. Blocking and permeabilization were performed for 30 min in DPBS with 5% donkey serum, 0.5% Triton-X100 and 0.05% BSA. Primary ab incubation (VMAT2, 1:50, EB06558, Everest Biotech, Ramona, USA, HA-tag: 1:50, 682404, Biolegend, San Diego, USA) was performed for 60 min, followed by Cy3 conjugate secondary ab incubation (1:250, kindly provided from Birgit Fehrenbacher, Department of Dermatology, University of Tuebingen, Germany). Coverslips were mounted using ProLong Antifade Diamond Medium containing DAPI (LifeTechnologies, Massachusetts, USA), and imaged with a TCS-SP2/Leica DM IRE2 confocal laser scanning microscope. Images were processed with Leica Confocal Software LCS (Version 2.61) (original magnification 630) (Leica Microsystems, Wetzlar, Germany).

*Stereotactic injections*

Rats were anesthetized by injecting a mixture (1 mL/kg) of fentanyl (0.005 mg/kg), midazolam (2 mg/kg) and medetomidine (0.15 mg/kg) intraperitoneally, and were placed onto a stereotactic frame (Harvard Apparatus, Holliston, MA, USA) with the skull flat between Lambda and Bregma. The following coordinates were used for the injections (flat skull position): AP: - 5 mm, ML: ± 2 mm, DV: - 7.2 mm, below dural surface as calculated relative to Bregma. Rats were injected with 3 μL AAVs into the right SNc and were divided in two groups. Control rats (n= 10) were injected with AAV-PHP.EB-sgRNA-*LacZ* (1.7 x 10$^{12}$gc/mL) and AVV-PHP.EB-SaCas9 (1.4 x 10$^{12}$ gc/mL) (1:1 ratio). VMAT2 KD rats (n= 45) were injected with AAV-PHP.EB-sgRNA-*Slc18a2* (2.1 × 10$^{12}$ gc/mL) and AVV-PHP.EB-SaCas9 (1.4 x 10$^{12}$ gc/mL) (1:1 ratio) (see Fig. 1a for vector constructs). DPBS (3 μL) was injected into the contralateral side to control for the sham lesion. Solutions were infused at a rate of 0.2 μL/min using a 5 μL Hamilton syringe (Hamilton, Bonaduz, Switzerland) and an automated microsyringe pump (Harvard Apparatus, Holliston, MA, USA). To allow for the diffusion of AAVs into the tissue, and prevent the solution from flowing backwards, the needle was left in place for 10 min, and then slowly retracted at 0.2 mm (DV)/min. After the surgery an antidote containing ati-pamezole (0.75 mg/kg) and flumazenil (0.2 mg/kg) was injected subcutaneously. The rats were kept warm in their cages until fully recovered.

*Radiotracer synthesis*

[$^{11}$C]Dihydrotetrabenazine (DTBZ) was synthetized starting from [$^{11}$C]-iodomethane, which was reacted with (+)- 9-O-desmethyl-dihydrotetrabenazine in the presence of base using the "loop" method [38]. Afterwards, it was purified by HPLC, and formulated as a sterile pyrogen-free saline solution. The total synthesis time from the end of the beam was 50 min. The radiochemical purity of the final radiotracer was > 95% as determined by HPLC. Molar activity (MA) was determined at the time of injection and ranged between 96 ± 37 GBq/μmol.

[$^{11}$C]Raclopride (RAC), a D2 receptor ligand, was synthesized by alkylation of S-(+)-O-desmethyl-raclopride (ABX, Radeberg, Germany) using [$^{11}$C]methyl triflate. Therefore, high SA [$^{11}$C]CH$_3$I was prepared using the automated Tracerlab FX Mel module (GE Healthcare, Uppsala, Sweden) and converted to [$^{11}$C]methyl triflate using the online AgOTf column oven of the module. Radiolabeling was performed in a Tracerlab FX M synthesizer for $^{11}$C-methylations (GE Healthcare, Uppsala, Sweden) along standard procedures established within the department. After purification and formulation, the product was obtained with a 13 ± 5% decay-corrected radiochemical yield (from [$^{11}$C]methyl triflate). The total synthesis time from the end of the beam was 50 min. The radiochemical purity of the final formulated radiotracer was > 95% as determined by HPLC. MA was determined at the time of injection and was calculated as 88 ± 41 GBq/μmol.

D-threo-[$^{11}$C]Methylphenidate (MP), a DAT ligand, was synthesized by alkylation of D-threo-N-NPS-ritalinic acid (ABX, Radeberg, Germany) using [$^{11}$C]methyl iodide (CH$_3$I) [39]. High specific activity (SA) [$^{11}$C]CH$_3$I was prepared using a Tracerlab FX Mel module (GE Healthcare, Uppsala, Sweden). The radiolabeling was performed in a PET tracer synthesizer for $^{11}$C-methylations (Tracerlab FX M). After purification and formulation, the product was obtained with a 41 ± 13% decay-corrected radiochemical yield (from [$^{11}$C]MeI). The total synthesis time was 60 min, and the radiochemical purity of the final formulated radiotracer was > 95% as determined by HPLC analysis. MA was determined at the time of injection as 58 ± 14 GBq/μmol.

[$^{18}$F]GE-180 was synthetized on a FASTlab synthesizer (GE Healthcare, Uppsala, Sweden) with the single-use cassettes. The fully automated system is designed for single-step fluorinations with cyclotron-produced [$^{18}$F]fluoride via the $^{18}$O($p,n$)$^{18}$F nuclear reaction on a PETtrace Mel Microlab module (GE Healthcare, Uppsala, Sweden). Irradiations were performed using a dual-beam, 30-μA current on two equal Ag targets with HAVAR foils using 16.5 MeV protons. Each target contained 1.6 mL of ≥ 96% [$^{18}$O]water. The total synthesis time from the end of the beam was 50 min. The radiochemical purity of the final formulated radiotracer was > 95% as determined by HPLC. MA was determined at the time of injection and ranged between 576 ± 283 GBq/μmol.

For [$^{11}$C]flumazenil, 2 mg desmethyl flumazenil were dissolved in 0.3 ml DMF with 3 μl 5 M NaOH and reacted for 2 min at 80 °C. After reaction the products were purified by semi-preparative HPLC ([$^{11}$C]DASB: Luna C18(2), Phenomenex, Aschaffen-burg, Germany; 3 mM sodium hydrogen phosphate in 64% acetonitrile, [$^{11}$C]Flumazenil: Su-pelcosil ABZ+Plus, Sigma-Aldrich, Steinheim, Germany; 8 mM phosphoric acid in 16% acetonitrile) and reformulated by solid-phase extraction (Strata-X, Phenomenex; elution with 0.5 ml ethanol, dilution with 5 ml phosphate-buffered saline). The radiochemical purity of the final formulated radiotracer was > 95% as determined by HPLC. MA was determined at the time of injection as109.5 ± 39.6 GBq/μmol.

*In vivo PET imaging and data analysis*

VMAT2 KD (n= 14) and control rats (n= 10) underwent 60-minutes dynamic PET emission scans with [$^{11}$C]DTBZ (8 weeks post-injection), [$^{11}$C]MP (10 weeks post-injection), [$^{11}$C]RAC (12 weeks post-injection) and [$^{18}$F]GE-180 (14 weeks post-injection). Four scans were excluded from all analyses because two animals from each cohort died during the PET acquisition. One control rat was excluded from the [$^{11}$C]MP analysis due to major artefacts.

Three small-animal PET scanners (Inveon, Siemens, Erlangen, Germany) and dedicated rat brain beds (Jomatik Gmbh, Tuebingen, Germany) with stereotactic holders and temperature feedback control units (Medres, Cologne, Germany) were used. These ensured the delivery and removal of the anesthesia gas and stabilized the body temperature at 37 °C during the PET data acquisition. Animals were anesthetized with 2% isoflurane vaporized in 1.0 L/min of oxygen. Subsequently, a 24 G catheter (BD Insyte, NJ, USA) was placed into the tail vein for the tracer administration. The rats were placed in the center of the field of view (FOV) and PET acquisitions started 5 s prior to the bolus injection of the tracer. In Table 2 injected activity (MBq/kg) and MA (GBq/μmol) at the time of injection are reported for each radioligand. The list mode data from the dynamic acquisitions of [$^{11}$C]DTBZ, [$^{11}$C]MP, and [$^{11}$C]RAC were histogrammed into 39 time frames (12x5 s, 6x10 s, 6x30 s, 5x60 s, 10x300 s), from [$^{18}$F]GE-180 into 16 time frames (5x60 s, 5x120 s, 3x300 s, 3x600 s), and reconstructed using OSEM3D map algorithm and a matrix size of 256 x 256, resulting in a pixel size of 0.38 x 0.38 x 0.79 mm.

Table 2: [$^{11}$C]DTBZ, [$^{11}$C]MP, [$^{11}$C]RAC and [$^{18}$F]GE-180 injected and molar activities

| Radioligand | Injected Activity (Mean ± SD) MBq/Kg | Molar Activity (Mean ± SD) GBq/μmol |
|---|---|---|
| [$^{11}$C]DTBZ | 25.0 ± 1.3 | 93.1 ± 34.9 |
| [$^{11}$C]MP | 25.5 ± 1.4 | 57.5 ± 14.1 |
| [$^{11}$C]RAC | 25.5 ± 1.1 | 88.4 ± 41.2 |
| [$^{18}$F]GE-180 | 24.3 ± 2.7 | 576.4 ± 282.7 |

Data preprocessing analysis was performed with Matlab (Mathworks, Natick, MA, USA), Statistical Parametric Mapping 12 (SPM12, Wellcome Trust Centre for Neuroimaging, University College London, England) and the QModeling toolbox. First, realignment of all frames was performed using SPM12 and average images were generated for every scan. The mean images were then used for coregistration to the Schiffer rat brain atlas. To generate the respective time activity curves (TAC), volumes of interest (VOIs) were defined over the right and left striatum (target region) and the cerebellum (reference region). TACs were extracted for each scan and expressed as standardized uptake values (SUV):

$$SUV(t) = \frac{radioactivity\ concentration\ (MBq\ mL^{-1})}{injected\ dose\ (MBq)/body\ weight\ (Kg)}$$

Binding potentials (BP$_{ND}$) for [$^{11}$C]DTBZ, [$^{11}$C]RAC, and [$^{11}$C]MP were calculated over the all frames, in the right and left striatum using the Logan reference, with a population average k2' ([$^{11}$C]DTBZ: 0.41 min$^{-1}$, [$^{11}$C]RAC: 0.34 min$^{-1}$, [$^{11}$C]MP: 0.18 min$^{-1}$). [$^{11}$C]DTBZ, [$^{11}$C]RAC and [$^{11}$C]MP binding potential changes, here expressed as ΔBP$_{ND}$ (%), were calculated according to the formula:

$$\Delta BP_{ND}(\%) = \frac{1 - BP_{ND}\ right\ striatum}{BP_{ND}\ left\ striatum} \times 100$$

[$^{11}$C]DTBZ ΔBP$_{ND}$ was used as a surrogate measure of synaptic dysfunction, and to separate the VMAT2 KD rats into mild and moderate.

[$^{18}$F]GE-180 SUVs of the right striatum were calculated over the interval between 30 - 60 minutes after scan start and normalized by the SUVs calculated for the left (DPBS-injected) striatum over the same time interval.

QModeling was also used to generate voxel-wise BP$_{ND}$ maps for [$^{11}$C]DTBZ, [$^{11}$C]RAC, and [$^{11}$C]MP and average images for [$^{18}$F]GE-180.

*Simultaneous [$^{11}$C]RAC PET/fMRI experiments*

LE rats (n= 30) underwent [$^{11}$C]RAC PET/fMRI scans at baseline and 8 - 14 weeks after CRISPR/SaCas9 targeting. Seven scans were excluded from all analyses due to major artefacts, motion over the course of the scan, or because the animal died during the scan. The final cohort included 23 LE rats.

Anesthesia was induced by placing the animals in knock-out boxes and delivering 2% isoflurane in regular air. Subsequently, a 24 G catheter (BD Insyte, NJ, USA) was placed into the tail vein for the tracer and *i.v.* anesthesia administration. Afterwards, rats received a medetomidine bolus (0.05 mg/kg) and the anesthesia was switched to constant medetomidine infusion (0.1 mg/kg/h), and 0.5% isoflurane in air during the scan time. Next, rats were placed onto a dedicated rat bed (Medres, Cologne, Germany) with stereotactic holders and a temperature feedback control unit (Medres, Cologne, Germany), ensuring the delivery and removal of the anesthesia gas and stabilizing the body temperature at 36.5 °C during the scan time. A breathing pad and a pulse oximeter were used to observe respiration and heart rates. Scans were acquired using a small-animal 7 T Clinscan MRI scanner (Bruker Biospin MRI, Bruker, Ettlingen, Germany), a 72 cm diameter linearly polarized RF coil (Bruker) for transmission and a four-channel rat brain coil (Bruker) for reception. Localizer scans were first acquired to accurately position the rat brains into the center of the PET/MRI FOV. Subsequently, local field homogeneity was optimized by measuring local magnetic field maps. Anatomical reference scans were performed using T2-weighted Turbo-RARE MRI sequences (TR: 1800 ms, TE: 67.11 ms, FOV: 40 x 32 x 32 mm, image size: 160 x 128 x 128 px, Rare factor: 28, averages: 1). Finally, T2*-weighted gradient echo EPI sequences (TR: 2500 ms, TE: 18 ms, 0.25 mm isotropic resolution, FoV 25 x 23 mm, image size: 92 x 85 x 20 px, slice thickness 0.8 mm, slice separation 0.2 mm, 20 slices) were acquired for fMRI.

A dedicated small-animal PET insert developed in cooperation with Bruker was used for [11C]RAC acquisitions, the second generation of a PET insert developed in-house. [11C]RAC was applied via a bolus injection. In Table 3 injected activities (MBq/kg) and MA (GBq/μmol) at the time of injection are reported. PET/fMRI acquisitions started simultaneously to tracer injection and were performed over a period of 60 min. The list-mode files of the PET data were histogrammed into 60 1-minute time frames and reconstructed using an in-house written OSEM2D algorithm. Data preprocessing analysis was performed as described above (see paragraph *In vivo* PET imaging and data analysis). A population average k2' ([11C]RAC baseline: 0.20 min⁻¹, [11C]RAC VMAT2 KD: 0.23 min⁻¹) was set for the Logan reference.

Table 3: [11C]RAC, [11C]DTBZ, [11C]FMZ injected and molar activities

| Radioligand | Injected Activity (Mean ± SD) MBq/Kg | Molar Activity (Mean ± SD) GBq/μmol |
|---|---|---|
| [11C]RAC t0 | 26.8 ± 4.5 | 108.0 ± 49.3 |
| [11C]RAC t8 | 27.6 ± 0.9 | 115.5 ± 41.4 |
| [11C]DTBZ | 26.8 ± 0.9 | 180.5 ± 48.3 |
| [11C]FMZ | 27.1 ± 0.9 | 109.5 ± 39.6 |
| [11C]DASB | 25.8 ± 0.9 | 121.6 ± 73.1 |

Preprocessing of the fMRI data was performed using a pipeline employing SPM12, Analysis of Functional Neurolmages (AFNI, National Institute of Mental Health (NIMH), Bethesda, Maryland, USA) and in-house written scripts in MATLAB, as reported previously.

First, the PET and fMRI scans were realigned, and mean images were created with SPM 12. For fMRI, the realignment parameters were inspected and scans containing significant motion were excluded partially or completely from the analysis, as described above. Additionally, the realignment parameters were stored for the nuisance regression performed later in the pipeline.

The mean images generated by the realignment process for all PET and fMRI scans were then used to build binary brain masks of the fMRI and PET datasets using AFNI. Additionally, binary brain masks were generated for anatomical images and the respective masks were employed to remove extra-cerebral tissues from all scans. For fMRI, the masks were additionally used to generate a further set of images excluding the brain and containing solely the extra-cerebral tissues. The first ten principal components (PC) of the signal from these tissues were extracted using AFNI and stored for later use as nuisance parameters for data cleaning. Preprocessing was then continued in SPM 12 by first co-registering the realigned and skull-stripped PET and fMRI datasets to their anatomical references. The anatomical scans were then used to normalize all datasets to the Schiffer rat brain atlas. Following normalization, nuisance regression was performed using the 6 motion parameters and 10 PCs stored previously. Finally, a 1.5 x 1.5 x 1.5 mm³ full-width-half-maximum (FWHM) Gaussian kernel corresponding to the resolution of the PET insert was applied for spatial smoothing of all PET and fMRI datasets.

Resting-state functional connectivity was calculated using a seed-based approach. To this extent, 20 regions were selected from the Schiffer rat brain atlas (a list of the regions is provided in Table 4). The SPM toolbox Marseille Boîte À Region d'Intérêt (MarsBaR) was employed to extract fMRI time-courses from all regions. These were then used to calculate pairwise Pearson's r correlation coefficients for each dataset, generating correlation matrices containing 20 x 20 elements. Self-correlations were set to zero. The computed Pearson's r coefficients then underwent Fischer's transformation into z values for group-level analysis.

Table 4: Brain regions included in the Paxinos rat brain atlas, including their respective volumes and abbreviations. Data were analyzed with paired t-test

| Brain region (ROI) | Hemisphere | ROI volume [mm³] | Abbreviation |
|---|---|---|---|
| Caudate Putamen | left | 43.552 | CPu |
| | right | | |

(continued)

| Brain region (ROI) | Hemisphere | ROI volume [mm$^3$] | Abbreviation |
|---|---|---|---|
| Cingulate Cortex | left | 14.480 | Cg |
| | right | | |
| Medial Prefrontal Cortex | left | 6.304 | mPFC |
| | right | | |
| Motor Cortex | left | 32.608 | M1 |
| | right | | |
| Orbitofrontal Cortex | left | 18.936 | OFC |
| | right | | |
| Parietal Cortex | left | 7.632 | PaC |
| | right | | |
| Retrosplenial Cortex | left | 18.920 | RS |
| | right | | |
| Somatosensory Cortex | left | 71.600 | S1 |
| | right | | |
| Anterodorsal Hippocampus | left | 25.064 | CA1 |
| | right | | |
| Thalamus | left | 30.712 | Th |
| | right | | |

Several rs-FC metrics were computed on different regional levels to investigate potential effects of D2R availabilities. Regional node strengths were computed as the sum of all correlations of one seed to the regions included in one network. Inter-regional node strengths were defined as the sum of the correlations of one node to the regions of another network. Network strengths were defined as the sum of strengths of all correlations between regions belonging to a network. Inter-network strengths were calculated as the sum of all correlations between two sets of regions belonging to two networks.

*In vivo PET imaging of DA-GABA interplay*

VMAT2 KD rats (n= 23), already scanned with PET/fMRI, underwent 60 min dynamic PET emission acquisitions with [11C]DTBZ (8 weeks post-injection), and [11C]FMZ (10 weeks post-injection). In Table 3 injected activities (MBq/kg) and MA (GBq/$\mu$mol) at the time of injection are reported for each radioligand. PET scans and data analysis were performed as described above (see paragraph *In vivo* PET imaging and data analysis). However, the anesthesia and reconstruction algorithm were kept consistent between the PET and PET/fMRI studies, performed on the same cohort, to avoid data misinterpretation.

The following target regions were analyzed: right and left striatum for [11C]DTBZ, right and left hippocampus, auditory/visual/parietal/sensory/orbitofrontal cortex for [11C]FMZ. Cerebellum was used as reference region for [11C]DTBZ. Pons were used as reference region for [11C]FMZ.

A population average k2' ([11C]DTBZ: 0.42 min$^{-1}$, [11C]FMZ: 0.27 min$^{-1}$, [11C]DASB: 0.15 min$^1$) was used for the Logan reference.

*Behavioral experiments*

*Cylinder*

Rats were placed individually inside a glass cylinder (19 cm $\varnothing$, 20 cm height). The test started immediately, and lasted 5 min. During the test session rats were left undisturbed and were videotaped with a camera located at the bottom-center of the cylinder to allow a 360° angle view. Paw touches were analyzed using a slow-motion video player (VLC software, VideoLan). The number of wall touches, contacts with fully extended digits, was counted. Data were analyzed as follows:

$$contralateral\ paw\ touches\ (\%\ to\ total) = \frac{contralateral\ touches}{total\ touches} \times 100$$

*Rotameter*

Rotational asymmetry was assessed using an automated rotameter system composed of four hemispheres (TSE Systems GmBH, Bad Homburg, Germany) based on the design of Ungerstedt and Arbuthnott. Rats were placed into an opaque half bowl (49 cm $\varnothing$; 44 cm height) and fixed to a moveable wire with a collar. The number of clockwise (CW) and counterclockwise (CCW) rotations (difference of 42.3 ° in their position) were automatically counted. Spontaneous rotation test lasted 5 min. Apomorphine-evoked rotational asymmetry was evaluated for 60 min after s.c. administration of apomorphine hydrochloride (0.25 mg/kg) dissolved in physiological saline containing 0.1% ascorbic acid (Sigma Aldrich, St. Louis, Missouri, USA). Two priming injections of apomorphine (1 week interval off-drug) were necessary to produce sensitization to the treatment. The program RotaMeter (TSE Systems GmBH, Bad Homburg, Germany) was used to acquire the data. Data were analyzed as follows:

$$turns/min = ccw\ rotation\ -\ cw\ rotations$$

*Body weight gain*

Rats body weight was measured before (week 0) and 14 weeks after CRISPR/SaCas9-targeting. Body weight gain was calculated as follows:

$$body\ weight\ gain\ (g) = \frac{weight\ at\ week\ 14 - weight\ at\ week\ 0}{weight\ at\ week\ 0} \times 100$$

*Beam Walk*

Rats were trained for 4 days to cross a built-in-house beam (1.7 cm width, 60 cm length, 40 cm height), and reach a cage with environmental enrichment. Rats had 5 trials to cross the beam with the reward resting time decreasing from 30 s to 10 s. On the test day (one week apart from the fourth day of training) rats were videotaped. The acquired videos were analyzed using a slow-motion video player (VLC software, VideoLan) and the amount of footslips (falls) was counted. Data were analyzed as follows:

$$contralateral\ footslips\ (\%\ to\ total\ steps) = \frac{contralateral\ footslips}{total\ steps} \times 100$$

*Open Field*

Rats were set in a rectangular box (TSE Systems GmBH, Bad Homburg, Germany) for 11 min (1 min habituation, 10 min test), to evaluate the exploratory behavior. A frame with light sensors, as high as the animals' body center, was connected to a receiver box to record the rats' walked distance. The program ActiMod (TSE Systems GmBH, Bad Homburg, Germany) was used for the analysis and the experimental session was divided in 1 min time bins. Results were averaged from the total travelled distance over 10 min (mean $\pm$ SD).

*Statistics*

Statistical analysis was performed with GraphPad as follows: paired t-test was used for the statistical comparison within subjects, and unpaired t-test was used for the statistical comparison between groups. Correlations were inspected applying linear regression analysis. Synaptic dysfunction discrimination was tested with multiple-comparison Anova.

Results were considered significant for $^+P \leq 0.05$, $*P \leq 0.01$, $**P \leq 0.001$ and *ns* for P> 0.05. Values are detailed in legends, and results paragraph.

For the PET/fMRI data, inter-individual correlations between regional $BP_{ND-norm}$ values and rs-FC metrics were calculated using Pearson's r. This procedure was performed between each regional $BP_{ND-norm}$ and every rs-FC metric described above to determine potential modulations of edges, regional node strengths, global node strengths as well as intra-network and inter-network strengths by regional D2R densities. Additionally, each calculated correlation between $BP_{ND-norm}$ and rs-FC was tested for statistical significance.

## 2. Results

*CRISPR/SaCas9 gene-editing in rat primary cortical neurons*

The inventors generated five AAV-sgRNAs targeting the *Slc18a2* gene, and screened their knockdown (KD) efficiency *in vitro.* Rat primary cortical neurons were co-transduced with the AAV-sgRNAs and AAV-SaCas9 and the KD efficiency was evaluated on DNA (Figure 1) and protein level (Figure 2).

Seven days post-transduction, genomic DNA was extracted and analyzed with Surveyor assay. This methods allows the recognition of small Insertions/Deletions (InDel) using an enzyme which cleaves the mismatched sites in both DNA strands with high specificity (see Figure 1A for the assay procedure).

Fragments of the expected size were obtained for the sgRNA-*Slc18a2*. The remaining sgRNAs (2.2-2.5) showed no InDel formation (Figure 1B).

Seven days post-transduction, immunofluorescence was performed to assess the VMAT2 protein expression levels. Immunofluorescence of nucleic acids in the cell nucleus with DAPI (first column), GFP (second column) and VMAT2 expression (third column) in cultured rat cortical neurons is shown in Figure 2.

In non-transduced neurons (Figure 2A) and neurons transduced with the control sgRNA, targeting the bacterial *LacZ gene* (Figure 2B), VMAT2 expression was highly abundant in the cytoplasm. In contrast, in neurons transduced with sgRNA-*Slc18a2* the inventors observed a detectable KD of the VMAT2 protein (Figure 2C). In those samples, the GFP positive neurons, showed a visible reduction of the VMAT2 staining (arrows). Neurons transduced with sgRNAs 2.2-2.5 did not show reduction of VMAT2 expression (Figure 2D-G).

SgRNA-*Slc18a2* was the only sgRNA to induce appreciable InDel mutation rate and protein suppression, and was therefore selected to conduct the *in vivo* studies.

To evaluate the efficiency of the AAV-based CRISPR/SaCas9 KD in rat primary neurons, the inventors generated AAV-SaCas9, and AAV-sgRNA targeting the first exon of the bacterial *LacZ* (Control) gene or the second exon of the *Slc18a2* gene (Fig. 3a). Seven days post-transduction, the protein expression levels were inspected performing immunofluorescence (IF) (Fig. 3b). IF indicated a clear reduction of VMAT2 protein expression in neurons transduced with AAV-sgRNA-*Slc18a2* and AAV-SaCas9 (Fig. 3c).

*In vivo PET imaging* of *CRISPR/SaCas9 gene-editing in the adult rat brain*

To test the *in vivo* efficiency of the CRISPR/SaCas9 gene-editing, the inventors injected AAV-SaCas9 and AAV-sgRNA (*Slc18a2* or *LacZ*) into the right substantia nigra pars compacta (SNc) of adult rats, DPBS was injected into the left SNc. PET scans were performed 8 - 14 weeks post-injection (Fig. 4a).

*In vivo* [11C]DTBZ PET imaging was carried out to quantify the VMAT2 striatal content. The inventors observed ~ 30% decrease of [11C]DTBZ $BP_{ND}$ in the right striatum of rats injected with AAV-sgRNA-*Slc18a2* (VMAT2 KD), while no changes were detected in control rats. Compensatory VMAT2 changes did not occur in the contralateral striatum (DPBS-injected), as [11C]DTBZ $BP_{ND}$ did not differ between the left striatum of control and VMAT2 KD rats ([11C]DTBZ $BP_{ND}$: control: right striatum: $3.0 \pm 0.3$, left striatum: $2.9 \pm 0.3$, *n*= 8; *ns P*= 0.54; VMAT2 KD: right striatum: $1.9 \pm 0.7$, left striatum: $2.9 \pm 0.3$, *n*= 12; $**P$= 0.0003) (Fig. 4b,c, first row).

To investigate the decrease of DA levels in the striatum following the CRISPR/Cas9-induced VMAT2 KD in nigrostriatal neurons, the inventors performed [11C]RAC PET measurements and quantified the striatal D2R binding. The inventors observed increased binding of [11C]RAC in the right striatum of VMAT2 KD rats and no changes in control rats ([11C]RAC $BP_{ND}$: control: right striatum: $2.4 \pm 0.2$, left striatum: $2.4 \pm 0.3$, *n*= 8; *ns P*= 0.97; VMAT2 KD: right striatum: $2.6 \pm 0.4$, left striatum: $2.2 \pm 0.2$, *n*= 12; $**P$= 0.001) (Fig. 4b,c, second row), indicating a reduction of synaptic DA levels and/or compensatory changes of D2R expression at postsynaptic medium spiny neurons. Linear regression analysis identified a strong correlation between [11C]RAC $\Delta BP_{ND}$ and [11C]DTBZ $\Delta BP_{ND}$ ($R^2$= 0.64, $**P$< 0.0001) (Fig. 4d). To explore the threshold at which the observed postsynaptic changes occur, the inventors calculated the [11C]RAC/[11C]DTBZ binding ratio for the right and left striatum. The ratio remained close to 1 in control rats and in the DPBS-injected striatum, indicating no substantial difference between the two hemispheres. In contrast, VMAT2 KD rats displayed [11C]RAC $\Delta BP_{ND}$ when the level of synaptic dysfunction reached ~ 20%. From this point, a prominent increase in D2R densities/bindings was observed in the right striatum (Left striatum: $R^2$= 0.02, *ns P*= 0.59; right striatum: $R^2$= 0.78, ***P*< 0.0001) (Fig. 4e). This threshold was therefore set to separate the subjects into mild (n= 3) ([$^{11}$C]DTBZ $\Delta BP_{ND}$ < 20% (6.1 $\pm$ 7.8)), and moderate *(n=* 9) ([$^{11}$C]DTBZ $\Delta BP_{ND}$ > 20% (41.8 $\pm$ 17.3)). Notably, [$^{11}$C]RAC PET imaging was able to discriminate different degrees of synaptic dysfunction, classified from [$^{11}$C]DTBZ $\Delta BP_{ND}$ (Control vs mild *ns P*= 0.07, mild vs moderate ***P*= 0.004, control vs moderate ***P*< 0.0001) (Fig. 4f).

To inspect the integrity of dopaminergic nerve terminals and the occurrence of neuroinflammation after the VMAT2 KD, [$^{11}$C]MP PET imaging of the DAT and [$^{18}$F]GE-180 PET imaging of the activated microglia were performed. CRISPR/SaCas9 VMAT2 KD did neither affect [$^{11}$C]MP binding ([$^{11}$C]MP $BP_{ND}$: control: right striatum: 1.0 $\pm$ 0.1, left striatum: 0.9 $\pm$ 0.1, *n= 7; ns P*= 0.06; VMAT2 KD: right striatum: 0.9 $\pm$ 0.1, left striatum: 0.8 $\pm$ 0.1, *n*= 12; *ns P*= 0.82) (Fig. 4b,c, third row), nor [$^{18}$F]GE-180 uptake ([$^{18}$F]GE-180 $RSTR_{norm}$: control: 1.0 $\pm$ 0.1, *n*= 8; VMAT2 KD: 1.0 $\pm$ 0.1, *n*= 12; *ns P*= 0.17) (Fig. 4b,c, fourth row).

*Behavioral consequences of the CRISPR/SaCas9 gene-editing*

The inventors next analyzed the motor consequences of the CRISPR/SaCas9 gene-editing in VMAT2 KD and control rats.

To evaluate the locomotor asymmetry, the inventors performed the cylinder test, in which VMAT2 KD rats displayed a preference for the use of the right forepaw, while control rats exhibited a symmetric use of the forepaws (Contralateral paw touches, % to total: control: 50.5 $\pm$ 4.8, *n*= 8; VMAT2 KD: 37.8 $\pm$ 9.3, *n*= 12; ***P*= 0.002) (Fig. 5a). Paw use alterations highly correlated with [$^{11}$C]DTBZ $\Delta BP_{ND}$ ($R^2$= 0.71, ***P*< 0.0001), and [$^{11}$C]RAC $\Delta BP_{ND}$ ($R^2$= 0.67, ***P*< 0.0001) (Fig. 5b,c).

To assess the rotational behavior, the inventors performed the rotameter test with and without apomorphine administration. In the apomorphine-evoked test, VMAT2 KD rats exhibited more rotations to the contralateral side compared with control rats (CCW turns/min, APO-evoked: control: 4.2 $\pm$ 23.3; *n*= 8; VMAT2 KD: 237.8 $\pm$ 199.0; *n*= 12; ***P*= 0.0042) (Fig. 5d). Apomorphine-evoked rotations correlated with [$^{11}$C]DTBZ $\Delta BP_{ND}$ ($R^2$= 0.54, ***P*= 0.0002), and [$^{11}$C]RAC $\Delta BP_{ND}$ ($R^2$= 0.37, **P*= 0.005) (Fig. 5e,f). In the spontaneous rotation test, VMAT2 KD rats displayed a higher number of ipsilateral net turns compared with control rats (Turns/min: Control: - 5.6 $\pm$ 9.4, *n*= 8; VMAT2 KD: 6.7 $\pm$ 14.6, *n*= 12; $^+P$= 0.05) (Fig. 6a). The number of turns did not correlate with [$^{11}$C]DTBZ $\Delta BP_{ND}$ ($R^2$= 0.13, *ns P*= 0.12) and [$^{11}$C]RAC $\Delta BP_{ND}$ ($R^2$= 0.05, *ns P*= 0.33) (Fig. 6b,c).

Furthermore, the inventors inspected the VMAT2 KD impact on the rats' body weight gain. VMAT2 KD rats exhibited ~ 30% reduction in their gained weight over a period of 14 weeks, compared with controls (Body weight gain (g): control: 65.3 $\pm$ 13.2, *n*= 8; VMAT2 KD: 46.2 $\pm$ 6.7, *n*= 12; ***P*= 0.0004) (Fig. 5g). Body weight gain correlated with [$^{11}$C]DTBZ $\Delta BP_{ND}$ ($R^2$= 0.39, **P*= 0.003), and postsynaptic [$^{11}$C]RAC $\Delta BP_{ND}$ ($R^2$= 0.32, **P*= 0.01) (Fig. 5h,i).

Trained rats underwent the beam walk test to examine their motor function, coordination, and balance. VMAT2 KD rats stumbled with higher frequency to the left side, while control rats displayed equal chances to slip in each direction (Contralateral footslips, % to total steps: control: 55.3 $\pm$ 15.9, n= 8; VMAT2 KD: 78.7 $\pm$ 20.1, n= 12; **P*= 0.01) (Fig. 6d). No correlations between gait alterations and [$^{11}$C]DTBZ $\Delta BP_{ND}$ ($R^2$= 0.08, *ns P*= 0.24), or [$^{11}$C]RAC $\Delta BP_{ND}$ ($R^2$= 0.02, *ns P*= 0.51) were found (Fig. 6e, f).

The inventors observed a reduction in the locomotor activity of VMAT2 KD rats in the open field test (Travelled distance (m): control: 19.0 $\pm$ 1.7, n= 8; VMAT2 KD: 15.7 $\pm$ 4.2, n= 12; $^+P$= 0.03), not correlating with [$^{11}$C]DTBZ $\Delta BP_{ND}$ ($R^2$= 0.11, *ns P*= 0.14), and [$^{11}$C]RAC $\Delta BP_{ND}$ ($R^2$= 0.03, *ns P*= 0.44) (Fig. 6).

*CRISPR/SaCas9 gene-editing and [$^{11}$C]RAC PET/fMRI*

A second cohort of LE rats underwent [$^{11}$C]RAC PET/fMRI scans at baseline and after CRISPR/SaCas9 targeting, to inspect whole-brain FC changes following the VMAT2 KD. Within this study, in *vivo* [$^{11}$C]DTBZ PET scans and cylinder test were performed to verify the reproducibility of the synaptic and motor dysfunction in the VMAT2 KD animal model.

*In vivo* [$^{11}$C]DTBZ PET imaging was performed 8 weeks after CRISPR/SaCas9 targeting. The inventors observed ~ 20% decrease of [$^{11}$C]DTBZ $BP_{ND}$ in the right striatum of VMAT2 KD rats ([$^{11}$C]DTBZ $BP_{ND}$: VMAT2 KD: right striatum: 1.7 $\pm$ 0.3, left striatum: 2.0 $\pm$ 0.3, *n*= 23; ***P*< 0.0001) (Fig. 7a).

PET analysis of [$^{11}$C]RAC PET/fMRI scans, performed at baseline and 8 - 14 weeks following the VMAT2 KD, indicated increased ligand binding in the right striatum of VMAT2 KD rats and no changes at baseline ([$^{11}$C]RAC $BP_{ND}$: baseline: right striatum: 2.1 $\pm$ 0.3, left striatum: 2.1 $\pm$ 0.3, *n*= 23; *ns P*= 0.97; VMAT2 KD: right striatum: 2.3 $\pm$ 0.3, left striatum: 2.1 $\pm$ 0.2, *n*= 23; ***P*= 0.0001) (Fig. 7a). [$^{11}$C]RAC $\Delta BP_{ND}$ correlated with [$^{11}$C]DTBZ $\Delta BP_{ND}$ ($R^2$= 0.21, $^+P$= 0.03) (Fig. 7a). [$^{11}$C]RAC/[$^{11}$C]DTBZ binding ratio for the right and left striatum indicated a prominent increase in D2R densities/bindings in the right striatum when the level of synaptic dysfunction reached ~ 20% (Left striatum: $R^2$= 0.02, *ns P*= 0.49; right striatum: $R^2$= 0.52, ***P*< 0.0001) (Fig. 7a). According to this threshold VMAT2 KD rats were separated into mild (n= 13) ([$^{11}$C]DTBZ $\Delta BP_{ND}$ < 20% (6.2 ± 8.4)), and moderate (n= 10) ([$^{11}$C]DTBZ $\Delta BP_{ND}$ > 20% (32.7 ± 9.3)).

Rats' motor function, as assessed in the cylinder test, diminished of ~ 11% 12 weeks after VMAT2 KD (Contralateral paw touches, % to total: baseline: 50.0 ± 3.9, *n*= 23; VMAT2 KD: 44.0 ± 5.8, *n*= 23; **P*= 0.001) (Fig. 8a). Rats' performance in the cylinder test (% change from baseline) correlated with [$^{11}$C]DTBZ $\Delta BP_{ND}$ (R$^2$= 0.36, *P*= 0.003), but not with [$^{11}$C]RAC $\Delta BP_{ND}$ (R$^2$= 0.15, *ns P*= 0.07) (Fig. 8b,c).

*In vivo* PET data, together with the behavioral examination of the motor function, confirmed the reproducibility of the VMAT2 KD animal model.

The inventors next assessed the occurrence of FC changes in DMN and SMN following the VMAT2 KD. The inventors' analysis focused on identifying patterns of synaptic dysfunction spreading, and biomarkers of mild dysfunction.

Figure 7b,c illustrate intraregional FC group level correlation matrices at baseline (below matrix diagonal) and after VMAT2 KD (above matrix diagonal) in mild and moderate rats. Brain graphs, right to the matrices, display the nodes and edges (raw values) that demonstrated FC changes to baseline (%) in DMN (b) and SMN (c).

The inventors found minor intraregional FC alterations in VMAT2 KD mild rats (left panels), and high intraregional FC changes in VMAT2 KD moderate rats (right panels), in both DMN and SMN (% change group level correlation matrices are reported in Fig. 9a,b).

Specifically, VMAT2 KD mild rats revealed FC changes in regions of the anterior DMN, compared with baseline. Interestingly, converse FC alterations were found in the left mPFC and OFC, 18% FC decrease (*P*= 0.02), and right mPFC and Cg, 24% FC increase (*P*= 0.03). FC between regions of the anterior and posterior DMN was inspected and FC changes were found solely within the left OFC and RS, 21% FC decrease (*P*= 0.05) (Fig. 4b, left panel).

VMAT2 KD moderate rats exhibited increased FC in most regions of the anterior DMN such as left OFC and right Cg (50%, *P*= 0.01), and right mPFC (70%, *P*= 0.01). Of particular note, no FC changes were seen within the left OFC and RS in VMAT2 KD moderate rats (*ns P*= 0.13), yet FC alterations extended to other regions of the posterior DMN. The inventors found a 44 to 63% increase in FC within the right mPFC and all regions of the posterior DMN bilaterally, and increased FC within right RS and CA1 bilaterally (30%; *P*= 0.03; *P*= 0.04), and left PaC (40%; *P*= 0.03) (Fig. 7b, right panel).

In the SMN, VMAT2 KD mild rats revealed increased FC throughout the left S1 and Th, compared with baseline (17%; *P*= 0.04) (Fig. 7c, left panel).

FC increase throughout these regions doubled to 34% (*P*= 0.001) in VMAT2 KD moderate rats, and extended to all regions of the SMN except M1 and S1 bilaterally, right CPu and right S1, and M1 bilaterally (Fig. 7c, right panel).

Moreover, the inventors inspected internetwork FC changes at baseline and after the CRISPR/SaCas9 targeting. Figure 7d,e illustrate internetwork FC group level correlation matrices at baseline (above matrix) and after VMAT2 KD (below matrix) in mild (d) and moderate (e) rats. Brain graphs, right to the matrices, display the nodes and edges (raw values) that demonstrated internetwork FC changes to baseline (%).

The inventors found that FC alterations between DMN and SMN would occur regardless of the VMAT2 KD extent (% change group level correlation matrices are reported in Fig. 9c,d).

Rats with mild VMAT2 KD presented converse FC alterations between regions of the anterior/posterior DMN and the SMN, compared with baseline. A 30 to 60% increase in FC was observed between regions of the anterior DMN and the SMN. Specifically, between OFC and CPu, S1 bilaterally, between right mPFC and M1, S1 bilaterally. Instead, a 10 to 20% decrease in FC was found between posterior regions of the DMN and the SMN. Specifically, between left RS and right M1 (*P*= 0.04), and S1 bilaterally (*P*= 0.006, *P*= 0.05) (Fig. 7d).

VMAT2 KD moderate rats presented increased FC between regions of the anterior/posterior DMN and the SMN, compared with baseline. Of particular note, internetwork FC changes were not found between the regions of the posterior DMN and the SMN that showed decreased FC in the mild rats. Moreover, between-network FC increase exacerbated in regions which appeared already altered in mild rats, and extended to others. A 20 to 30% increase in FC was found *f.e* between the right Cg and M1 bilaterally (*P*= 0.03, *P*= 0.02) and S1 bilaterally (*P*= 0.04), and between CA1 and left CPu (*P*= 0.02), left S1 (*P*= 0.004, *P*= 0.008), and M1 bilaterally (*P*= 0.03, *P*= 0.05). FC increased above 50% between the left Th and mPFC bilaterally (*P*= 0.02, *P*= 0.03). Importantly, FC increase exacerbated to ~ 60% between the right mPFC and M1 bilaterally (*P*= 0.01, *P*= 0.003), and to ~ 80% between the right mPFC and S1 bilaterally (*P*= 0.001, *P*= 0.003) (Fig. 7e).

To complement the results of the intraregional and internetwork FC (group level correlation matrices including both are displayed in Supplementary Fig. 9e,f), the inventors evaluated changes of regional mean connection distances. Network-wise graph theoretical analysis on node level was paralleled by whole-brain connection-wise analysis to identify the nodes that were significantly altered in VMAT2 KD mild and moderate rats, compared with baseline, for the DMN (Fig 7f) and SMN (Fig 7g). Briefly, the network organization did not change in VMAT2 KD mild rats, compared with baseline, as changes in the global mean connection distance were not found in DMN (Fig. 7f, left panel) nor SMN (Fig. 7g, left panel). On the contrary, graph theoretical analyses revealed that rats with moderate VMAT2 KD, presented shorter functional paths in nodes of both DMN and SMN. Interestingly, while network organization changes affected bilaterally the regions of the DMN (Fig. 7f, right panel), they occurred to a higher extent in the left regions of the SMN (Fig. 7g, right panel).

*CRISPR/SaCas9 gene-editing and in vivo PET imaging of DA-GABA interplay*

Following the *in vivo* PET/fMRI evaluation of the FC changes, VMAT2 KD rats (n= 23) underwent PET scans with the GABA-A radioligand [11C]FMZ, to inspect the effects of the CRISPR/SaCas9 editing beyond the targeted dopaminergic neurotrans-mission.

*In vivo* [11C]FMZ PET imaging was carried out 10 weeks post-CRISPR/SaCas9-targeting to quantify the GABA-A binding in the hippocampus and the auditory, visual, parietal, sensory, orbitofrontal cortices.

The inventors observed a reduction of [11C]FMZ $BP_{ND}$ in the right CA1 (~11%), Au (~ 12%), V1 (~ 9%), PaC (~ 11%) and S1 (~ 5%). Interestingly, we observed an increase of [11C]FMZ $BP_{ND}$ in the right OFC (~ 6%) ([11C]FMZ $BP_{ND}$: right hippocampus: $0.55 \pm 0.09$, left hippocampus: $0.62 \pm 0.10$, **$P$= 0.001; right auditory cortex: $0.70 \pm 0.09$, left auditory cortex: $0.79 \pm 0.09$, **$P$= 0.001; right visual cortex: $0.75 \pm 0.08$, left visual cortex: $0.82 \pm 0.11$, *$P$= 0.003; right parietal cortex: $0.71 \pm 0.09$, left parietal cortex: $0.79 \pm 0.10$, **$P$= 0.001; right sensory cortex: $0.88 \pm 0.09$, left sensory cortex: $0.92 \pm 0.09$, *$P$= 0.003; right orbitofrontal cortex: $0.80 \pm 0.15$, left orbitofrontal cortex: $0.75 \pm 0.12$, **$P$= 0.001; *n*= 23; Bonferroni-Sidak corrected $P$ values) (Fig. 10a,b).

Notably, [11C]FMZ $BP_{ND}$ reduction/increase was not conditional on the degree of synaptic dysfunction, estimated from [11C]DTBZ $\Delta BP_{ND}$ (Mild: [11C]DTBZ $\Delta BP_{ND}$ < 20%; Moderate: [11C]DTBZ $\Delta BP_{ND}$ > 20%). Indeed, [11C]FMZ $BP_{ND}$ data would be consistent either if the entire cohort was analyzed or if this was separated into VMAT2 KD mild (*n*= 13) (Fig. 10c), and moderate rats (*n*= 10) (Fig. 10d). This was further evidenced by lack of correlation between [11C]DTBZ $\Delta BP_{ND}$ and [11C]FMZ $\Delta BP_{ND}$ (linear regression data not shown).

### 3. Discussion

*In vitro experiments*

The inventors detected low InDel frequency *in vitro* possibly due to the genomic mosaicism and low transduction rate achieved in primary neurons, typically hard-to-transfect cells.

Next, the inventors examined the genome editing efficacy in downregu-lating protein expression. Interestingly, the inventors observed a heterogeneous population. Indeed, some neurons lacking the expression of AAV reporters, displayed intact protein expression, while GFP and HA-tag double-positive neurons showed either visible VMAT2 reduction, as expected, or intact protein signal. It might be possible that the repair following the Cas9 DSB fails to silence the gene, thus the transduced neurons will not acquire the KD phenotype. In brief, KD neurons may contain a mosaic of different genotypes. It is important to keep in mind that InDels may occur on one or both alleles of the target gene and the resulting InDels in a single neuron may differ from one another.

Another interesting observation is the detection of VMAT2 in cortical neurons. Despite not being found in the cortex of adult rats, the protein is transiently expressed in thalamocortical afferents from embryonic day E16 to postnatal stage P6.

*In vivo PET imaging*

The inventors used [11C]DTBZ PET imaging to assess the efficiency of the CRISPR/Cas9-induced VMAT2 KD *in vivo.* Indeed, the radioligand is a specific marker for VMAT2 and is widely used to estimate the severity of dopaminergic lesions. Being also sensitive to serotoninergic terminals, [11C]DTBZ is not an exclusive marker for DA, however 90 % of monoaminergic innervation in the striatum is of dopaminergic origin. [11C]DTBZ data evidenced decreased $BP_{ND}$ in KD but not control rats (Fig. 4b,c, first row), indicating effective VMAT2 silencing. It should be emphasized that the terms VMAT2 silencing/KD refer to the density of VMAT2 binding sites, and not to VMAT2 function.

Synaptic dysfunction did not trigger compensatory effects in the contralateral striatum (DPBS-injected), contrasting with earlier observations in rat models of PD and suggesting that this type of compensation arises as a consequence of higher degrees of nigral loss.

The observed degree of synaptic dysfunction greatly varied among KD rats, in line with previous evidences of CRISPR/Cas9 editing in the rat brain, which efficiency ranged from ~ 20 to 70 % (IHC). The inventors speculate that the large range observed in our KD rats might be due to the diverse Cas9 cutting efficiency, rather than to the accuracy of the stereotactic injection. If that would be the case, the same deviation would be observed in control rats as well as into the contralateral hemisphere (DPBS-injected). Previous reports show slow kinetics of Cas9, which seems to adhere to DNA for several hours post-cutting, leading to variable rates of DSB repair. This is especially true for postmitotic neurons, where the persistent Cas9 expression and cleavage of any properly repaired sequence possibly leads to incorrect repair and inactivation on both alleles. Furthermore, the misexpressed truncated proteins resulting from the random repair at the DSB cleavage site may generate partial loss or gain-of-function phenotypes in non-dividing neurons, obscuring the readout. Moreover, the repair following the Cas9 DSB might fail to silence the gene. As a result of the genomic mosaicism, the CRISPR/Cas9-induced VMAT2 KD efficiency will be limited by the number of neurons within the population not acquiring the KD phenotype.

The CRISPR/Cas9-induced synaptic dysfunction was paralleled by postsynaptic compensatory changes, as evidenced from [$^{11}$C]RAC PET imaging (Fig. 4b,c, second row), in agreement with previous observations in human PD patients, toxin-lesioned primates and rats. This increase likely reflects a combination of synaptic DA depletion and D2R upregulation. A possible confounding factor might arise from the fact that the observed DAR changes are due to the nigrostriatal DA system differences between brain hemispheres. Indeed, studies performed on female intact animals have shown greater DAR binding (40 %) in the right striatum compared to the left striatum. However, the inventors can exclude this hypothesis in our animal model, since that DAR binding did not differ between striata of controls. The observed postsynaptic changes correlated with the degree of synaptic dysfunction, estimated from [$^{11}$C]DTBZ data (Fig. 4d), as previously reported in PD patients. A potential interesting finding is the substantial increase in [$^{11}$C]RAC BP$_{ND}$ in response to the moderate level of synaptic DA loss ($\sim$ 40 %) (Fig. 4e). Indeed, in previous experimental models of PD, DAR changes seem to appear as a consequence of severe denervation (70-90 % DA loss). Notably, [$^{11}$C]RAC could distinguish between three degrees of synaptic dysfunction, indicating its potential as an early diagnostic marker and its utility to discriminate and predict the extent of DA loss in PD (Fig. 4f).

Importantly, using CRISPR/Cas9 to KD the VMAT2 gene the inventors were able to manipulate the DA signaling, preserving DA neurons (DAn) structurally. Indeed, [$^{11}$C]MP dynamic PET scans in KD rats displayed intact nerve terminal density (Fig. 4b,c, third row), in line with studies performed on genetic mice models of VMAT2 depletion and rat models of synaptic disruption. Consistently with our results, human studies show changes in striatal DA content, detected with [$^{11}$C]DTBZ, not accompanied by DAT changes. Nevertheless, the synaptic DA loss the inventors achieved might not be sufficient to trigger DAT changes, which seem to appear when 90 % denervation is reached. Remarkably, as evidenced by linear regression analysis, VMAT2 was selectively reduced without affecting nerve terminal integrity, emphasizing that the observed reduction in [$^{11}$C]DTBZ BP$_{ND}$ is mainly linked to the selective VMAT2 KD rather than to neuronal loss (Fig. 4g).

Nevertheless, the inventors wished to exclude the occurrence of inflammatory responses arising from the surgical procedure, AAVs integration or Cas9 presentation. [$^{18}$F]GE-180 PET results suggest that glial activation might not be the source/product of DAn degeneration, highlighting the lack of inflammatory and humoral responses triggered by the Cas9, surgical procedure and dopaminergic synaptic dysfunction (Fig. 4b,c, fourth row). However, the inventors' data might be also indicative of a lack of detection of the TSPO response, limited by the resolution of current PET scanners or [$^{18}$F]GE-180 performance in models of lower neuroinflammation, which better resemble the human PD condition. Indeed, a relatively large change in TSPO density must occur in order to be detected *in vivo* with PET imaging. Accordingly, studies in PD human and non-human primates found no significant increase in TSPO binding in the striatum, and also a lack of correlation with disease progression and glial activation.

*PET/fMR imaging*

*Motor consequences of the CRISPR/Cas9-induced VMAT2 KD*

The inventors could show that our CRISPR/Cas9-induced VMAT2 KD rat is a suitable PD experimental model mimicking some important motor features of the disease.

Indeed, KD rats revealed motor alterations in terms of locomotor activity (open field test), forelimb lateralization (cylinder test), spontaneous and apomorphine-evoked rotations (rotameter test), and gait (beam walk test) (Fig. 5).

**Claims**

1. A 'single guide RNA' (sgRNA) molecule targeting the *Slc18a2* gene in a living being for use in the prophylaxis and/or treatment and/or examination of a disease, **characterized in that** the sgRNA molecule comprises a nucleotide sequence which is selected from the group consisting of SEQ ID NOS: 1-5.

2. The sgRNA molecule of claim 1, **characterized in that** said disease is associated with the 'vesicular monoamine transporter 2' (VMAT2), preferably selected from the group consisting of: disorders related to dopaminergic function, neuropsychiatric disorders, Parkinson's disease (PD), movement disorders, Huntington's disease, tardive dyskinesia schizophrenia, and addiction.

3. A vector comprising the sgRNA molecule of claim 1 or 2.

4. The vector of claim 3, **characterized in that** said vector is an adeno-associated vector (AAV), preferably with AAV-PhP.EB serotype.

5. The vector of claim 3 or 4, **characterized in that** said sgRNA is under the control of an U6 promoter.

6. A composition comprising the vector of any of claims 3-5.

7. The composition of claim 6, further comprising a vector encoding CRISPR associated protein 9 (Cas9), preferably *Staphylococcus aureus* CRISPR Cas9 (SaCas9).

8. The composition of claim 7, **characterized in that** said Cas9 is under the control of a CAG promoter.

9. The composition of any of claims 6-8, which is a pharmaceutical composition comprising a pharmaceutically acceptable carrier.

10. The composition of any of claims 6-9 for the prophylaxis and/or treatment and/or examination of a disease associated with the 'vesicular monoamine transporter 2' (VMAT2).

11. The composition of claim 10, **characterized in that** said disease is selected from the group consisting of neuropsychiatric disorders, Parkinson's disease (PD), movement disorders, Huntington's disease and tardive dyskinesia.

12. A nucleic acid molecule, preferably an RNA molecule, further preferably an sgRNA molecule, said molecule comprising a nucleotide sequence which is selected from the group consisting of SEQ ID NOS: 1-5.

13. A method *in vitro* for editing the *Slc18a2* gene in biological material including genetic material encoding said *Slc18a2* gene, comprising the step of introducing the sgRNA of any of claims 1-2, and/or said vector of any of claims 3-5, and or said composition of any of claims 6-11, and/or said nucleic acid molecule of claim 12 into said biological material, preferably said editing is made via CRISPR/Cas9 technology.

14. A method for the prophylaxis and/or treatment and/or examination of a disease associated with 'vesicular monoamine transporter 2' (VMAT2) in a living being, comprising a step of editing the *Slc18a2* gene in said living being by introducing the sgRNA of any of claims 1-2, and/or said vector of any of claims 3-5, and or said composition of any of claims 6-11, and/or said nucleic acid molecule of claim 13 into said living being, preferably said gene editing is made via CRISPR/Cas9 technology.

15. The method of claim 14, **characterized in that** said disease is selected from the group consisting of: disorders related to dopaminergic function, neuropsychiatric disorders, Parkinson's disease (PD), movement disorders, Huntington's disease, tardive dyskinesia, schizophrenia, and addiction.

Fig. 1

Fig. 2

Fig. 3

c)

Fig. 3

Fig. 4

Fig. 4

Fig. 4

Fig. 5

**Fig. 5**

Fig. 6

g) h) i) Fig. 6

Panel g) Open Field; bar chart, Travelled distance (m), Control / VMAT2 KD.

Panel h) Travelled distance (m) vs [$^{11}$C]DTBZ $\Delta BD_{ND}$; $R^2 = 0.12$ (ns).

Panel i) Travelled distance (m) vs [$^{11}$C]RAC $\Delta BD_{ND}$; $R^2 = 0.03$ (ns).

**Fig. 7**

Fig. 7

Fig. 7

Fig. 8

Fig. 9

Fig. 9

**Fig. 9**

Fig. 10

**EUROPEAN SEARCH REPORT**

Application Number

EP 21 18 0165

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Hunker Avery C: "Combinatorial strategies using CRISPR/Cas9 for gene mutagenesis in adult mice", <br><br> 3 February 2021 (2021-02-03), XP55867114, Retrieved from the Internet: URL:https://digital.lib.washington.edu/res earchworks/bitstream/handle/1773/45248/Hun ker_washington_0250E_20968.pdf?sequence=1& isAllowed=y [retrieved on 2021-11-29] * page 51; figure 20 * | 1-15 | INV. C12N15/113 <br><br> ADD. A61P25/00 |
| A | MARCIANO S ET AL: "PS06-062 Evaluation of CRISPr/SaCas9 induced gene knockdown in primary neurons and N27 cells", JOURNAL OF CEREBRAL BLOOD FLOW & METABOLISM, vol. 37, 1 April 2017 (2017-04-01), pages 445-505, XP055867134, US ISSN: 0271-678X, DOI: 10.1177/0271678X17695990 * pages 479-480 * | 1-15 | |
| A | WANG YALI ET AL: "Vesicular monoamine transporter 2 (Vmat2) knockdown elicits anxiety-like behavior in zebrafish", BIOCHEMICAL AND BIOPHYSICAL RESEARCH COMMUNICATIONS, ELSEVIER, AMSTERDAM NL, vol. 470, no. 4, 20 January 2016 (2016-01-20), pages 792-797, XP029413154, ISSN: 0006-291X, DOI: 10.1016/J.BBRC.2016.01.079 * figure 1 * | 1-15 | |

TECHNICAL FIELDS SEARCHED (IPC)

C12N

-/--

The present search report has been drawn up for all claims

| Place of search | Date of completion of the search | Examiner |
|---|---|---|
| The Hague | 30 November 2021 | Romano, Alper |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

## EUROPEAN SEARCH REPORT

Europäisches
Patentamt
European
Patent Office
Office européen
des brevets

Application Number

EP 21 18 0165

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | Marciano Sabina ET AL: "Combining CRISPR/Cas9 and brain imaging: from genes to molecules to networks", bioRxiv, 11 October 2021 (2021-10-11), XP055867125, DOI: 10.1101/2021.09.10.459766 Retrieved from the Internet: URL:https://www.biorxiv.org/content/10.1101/2021.09.10.459766v3.full.pdf [retrieved on 2021-11-29] * page 60; table 1 * ----- | | |

TECHNICAL FIELDS
SEARCHED      (IPC)

The present search report has been drawn up for all claims

| Place of search | Date of completion of the search | Examiner |
|---|---|---|
| The Hague | 30 November 2021 | Romano, Alper |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SEO, J.W. et al.** Positron emission tomography imaging of novel AAV capsids maps rapid brain accumulation. *Nat Commun,* 2020, vol. 11 (1), 2102
- **CHATTERJEE, D. et al.** Enhanced CNS transduction from AAV.PHP.eB infusion into the cisterna magna of older adult rats compared to AAV9. *Gene Ther,* 2021
- **DAYTON, R.D. et al.** More expansive gene transfer to the rat CNS: AAV PHP.EB vector dose-response and comparison to AAV PHP.B. *Gene Ther,* 2018
- **CHAN, K.Y. et al.** Engineered AAVs for efficient non-invasive gene delivery to the central and peripheral nervous systems. *Nat Neurosci,* 2017, vol. 20 (8), 1172-1179
- **WEI, Y. et al.** CRISPR/Cas9 with single guide RNA expression driven by small tRNA promoters showed reduced editing efficiency compared to a U6 promoter. *RNA,* 2017, vol. 23 (1), 1-5
- **SUN, H. et al.** Development of a CRISPR-SaCas9 system for projection- and function-specific gene editing in the rat brain. *Sci Adv,* 2020, vol. 6 (12), eaay6687
- **HU, Z. et al.** A compact Cas9 ortholog from Staphylococcus Auricularis (SauriCas9) expands the DNA targeting scope. *PLoS Biol,* 2020, vol. 18 (3), e3000686
- **KIM, E. et al.** vivo genome editing with a small Cas9 orthologue derived from Campylobacter jejuni. *Nat Commun,* 2017, vol. 8, 14500
- **IBRAHEIM, R. et al.** All-in-one adeno-associated virus delivery and genome editing by Neisseria meningitidis Cas9 in vivo. *Genome Biol,* 2018, vol. 19 (1), 137
- **JACKSON, K.L. et al.** Better Targeting, Better Efficiency for Wide-Scale Neuronal Transduction with the Synapsin Promoter and AAV-PHP.B. *Front Mol Neurosci,* 2016, vol. 9, 116
- Remington - The Science and Practice of Pharmacy. 2020